# EUROPÄISCHE PATENTSCHRIFT

(11) **EP 2 881 131 B1**
(45) Veröffentlichungstag und Bekanntmachung des Hinweises auf die Patenterteilung: **24.04.2019**
(21) Anmeldenummer: 13195948.8
(22) Anmeldetag: 05.12.2013
(51) Int. Cl.: A61M 5/20, A61M 5/315

(54) **Antriebs- und Dosiervorrichtung für eine Injektionsvorrichtung mit einer vorgespannten Antriebsfeder**
Drive and metering device for an injection device with a pre-tensioned drive spring
Dispositif d'entraînement et de dosage pour un dispositif d'injection doté d'un ressort d'entraînement précontraint

(43) Veröffentlichungstag der Anmeldung: 10.06.2015
(73) Patentinhaber: TecPharma Licensing AG, 3401 Burgdorf (CH)
(72) Erfinder: Streit, Ursina, 3322 Schönbühl (CH); Schenker, Susanne, 4900 Langenthal (CH); Hostettler, Patrick, 3415 Hasle (CH)
(74) Vertreter: SSM Sandmair

(56) Entgegenhaltungen:
- WO-A1-2008/031235
- WO-A1-2009/105910
- WO-A1-2010/105376
- WO-A1-2013/119132

## Beschreibung

Die Erfindung betrifft eine Antriebs- und Dosiervorrichtung für eine Injektionsvorrichtung zum Verabreichen bzw. Ausschütten eines flüssigen Produkts, insbesondere eines Medikaments, wie zum Beispiel Insulin zur Diabetestherapie. Im Besonderen betrifft die Erfindung auch eine Injektionsvorrichtung, welche eine solche Antriebs- und Dosiervorrichtung aufweist.

Der Begriff "Medikament" umfasst hier jede fließfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus dem Stand der Technik, wie zum Beispiel der WO 2008/031237 A1 ist ein Injektionsgerät mit einer Dosisanzeigetrommel und einer Antriebsfeder bekannt. Die Antriebsfeder ist eine Uhrenfeder, die spiralförmig aus einem bandförmigen Material gewickelt ist. Beim Einstellen der gewünschten Produktdosis wird die Feder mit einer rotatorischen Bewegung gespannt. Für die Dosisausschüttung wird durch Betätigen eines Betätigungsknopfs am proximalen Ende der Vorrichtung eine Kolbenstange der Vorrichtung mit der Feder gekoppelt, wodurch die Feder die in ihr gespeicherte Energie an die Kolbenstange abgeben kann, wodurch die Kolbenstange in Ausschüttrichtung bewegt wird. Zum Einstellen einer neuen Dosis wird die Feder durch Drehen des Dosierknopfs wieder vorgespannt usw. Dies wird so oft wiederholt, bis der Produktbehälter geleert ist.

Aus der US 5,104,380 A ist eine Injektionsvorrichtung mit einer Schraubenfeder bekannt, welche bei der Dosierung ebenfalls rotatorisch vorgespannt wird, so dass die Schraubenfeder als Torsionsfeder bezeichnet werden kann. Die vor jeder Produktausschüttung durch Drehen eines Drehknopfs vorgespannte Feder gibt ihre Energie zum Vortrieb der Kolbenstange an die Kolbenstange ab.

Die EP 2 644 217 A1 beschreibt eine Injektionsvorrichtung mit einer Dosisanzeige und einem Federantrieb, wobei eine zwischen einem Vortriebsglied und einem Widerlager wirkende und im Auslieferungszustand der Vorrichtung vorgespannte Feder mit so viel Energie vorgespannt ist, dass sie die aus dem Produktbehälter maximal ausschüttbare Produktmenge in mehreren Einzelausschüttungen ausschütten kann.

Die WO 2008/031235 A1 offenbart eine Antriebs- und Dosiervorrichtung nach dem Oberbegriff des Anspruchs 1.

Der Erfindung liegt die Aufgabe zugrunde, eine Antriebs- und Dosiervorrichtung für eine Injektionsvorrichtung anzugeben, die dem Verwender eine einfache Verwendung der Vorrichtung, insbesondere eine einfache Dosiseinstellung erlaubt und dennoch eine kompakte Bauweise ermöglicht.

Die Aufgabe wird durch die Merkmale des Anspruchs 1 gelöst. Vorteilhafte Weiterbildungen ergeben sich aus den abhängigen Ansprüchen, der Beschreibung und den Figuren.

Die Erfindung geht von einer Antriebs- und Dosiervorrichtung für eine Injektionsvorrichtung zum Verabreichen eines flüssigen Medikaments oder Produkts aus. Die Antriebs- und Dosiervorrichtung weist ein Gehäuse auf. Das Gehäuse ist vorzugweise hülsenförmig und/oder länglich ausgebildet. Das Gehäuse kann sich zum Beispiel entlang seiner Längsachse erstrecken.

Das Gehäuse kann optional einen Produktbehälter aufnehmen oder selbst den Produktbehälter bilden. Das Gehäuse kann ein- oder mehrteilig sein. Zum Bespiel kann das Gehäuse einen proximalen Gehäuseteil bilden, der im Wesentlichen die Antriebs- und Dosiervorrichtung bildet, d. h. einen Antriebs- und Dosiermechanismus umfasst oder aufweist. Das Gehäuse kann ferner einen Produktbehälterhalter aufweisen, der den Produktbehälter, wie zum Beispiel eine Karpule, aufnimmt und mit dem Gehäuse oder dem proximalen Gehäuseteil verbunden ist. Diese Verbindung kann derart sein, dass der Produktbehälterhalter und das Gehäuse oder das proximale Gehäuseteil nach dem Verbinden unlösbar, d. h. nur noch durch Zerstörung von Verbindungselementen lösbar ist. Eine solche Ausführung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, die Antriebs- und Dosiervorrichtung ggf. mehrfach zu verwenden, d. h. einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann optional eine Zeigeeinrichtung, insbesondere ein Fenster aufweisen, mittels der oder durch welches eine aktuell eingestellte Dosis, vorzugsweise von einer Dosisskala eines Dosiseinstellelements, welches mit der Vorrichtung optional vorhanden sein kann, ablesbar ist.

Die Antriebs- und Dosiervorrichtung umfasst ein Dosiseinstellglied, das zum Beispiel als Dosierknopf ausgebildet ist. Das Dosiseinstellglied ist vorzugsweise vom Verwender (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Für die Einstellung der auszuschüttenden oder zu verabreichenden Dosis wird das Dosierglied vorzugsweise vom Verwender ergriffen und relativ zu dem Gehäuse und insbesondere der optional vorhandenen Zeigeeinrichtung um eine Drehachse, die vorzugsweise der Längsachse der zum Beispiel länglich ausgestalteten Antriebs- und Dosiervorrichtung entspricht, verdreht. Das zum Beispiel hülsenförmige Dosiseinstellglied kann vorzugsweise axialfest mit dem Gehäuse verbunden, insbesondere entlang der Längsachse des Gehäuses verschiebefest sein, wodurch vorteilhaft die intuitive Handhabung der Vorrichtung durch den Verwender erleichtert wird, da er lediglich eine Drehbewegung des Dosierglieds für die Dosiseinstellung auszuführen braucht.

Die Antriebs- und Dosiervorrichtung umfasst ein in dem Gehäuse aufgenommenes Abtriebsglied, welches vorzugsweise als Kolbenstange ausgestaltet sein kann. Das insbesondere längliche Abtriebsglied erstreckt sich vorzugsweise entlang der Längsachse der Antriebs- und Dosiervorrichtung. Das distale Ende des Abtriebsglieds kann zum Beispiel einen tellerförmigen Flansch aufweisen. Das distale Ende des Abtriebsglieds ist so angeordnet, dass es auf einen Kolben eines Produktbehälters, der an der Antriebs- und Dosiervorrichtung befestigbar ist, insbesondere mittels des Produktbehälterhalters, wirken kann. Das Abtriebsglied kann den Kolben des Produktbehälters zur Ausschüttung des Produkts in dem Produktbehälter verschieben, wenn das Abtriebsglied relativ zu dem Gehäuse in die distale Richtung bewegt wird.

Die Antriebs- und Dosiervorrichtung umfasst ein Antriebsglied, welches relativ zu dem Gehäuse und vorzugsweise um die Längsachse drehbar ist und während der Produktausschüttung so mit dem Abtriebsglied gekoppelt ist, dass eine Drehung des Antriebsglieds bewirkt, dass das Abtriebsglied relativ zu dem Gehäuse in die distale Richtung bewegt wird.

Die Antriebs- und Dosiervorrichtung umfasst erfindungsgemäß eine rotatorische oder mit einem Drehmoment vorgespannte Antriebsfeder, die während der Einstellung der Produktdosis, insbesondere wenn ein Betätigungsglied unbetätigt ist, zwischen das Dosiseinstellglied und das Antriebsglied geschaltet ist, wobei das Dosiseinstellglied während der Einstellung der Produktdosis, insbesondere wenn das Betätigungsglied unbetätigt ist, verdrehfest mit dem Antriebsglied gekoppelt ist. Hierdurch wird vorteilhaft bewirkt, dass während der Einstellung der auszuschüttenden Produktdosis eine Drehung des Antriebsglieds relativ zu dem Dosiseinstellglied, vorzugsweise in eine erste und zweite Drehrichtung verhindert wird. Durch die erfindungsgemäße Anordnung der Antriebsfeder zwischen Dosiseinstellglied und Antriebsglied kann der Bereich um das Abtriebsglied platzsparender ausgelegt werden bzw. kann mehr Platz für evtl. zusätzliche Bauteile bereitgestellt werden, welche der Antriebs- und Dosiervorrichtung zum Beispiel weitere Funktionen verleihen. Da die vorgespannte Antriebsfeder während der Dosiseinstellung nicht weiter vorgespannt wird, wird es dem Verwender ermöglicht, eine Dosiseinstellung mit wenig Kraftaufwand vorzunehmen. Ein Spannen oder Entspannen der Antriebsfeder, die während der Einstellung der Produktdosis zwischen das Dosiseinstellglied und das Antriebsglied geschaltet ist, wird vorteilhaft dadurch verhindert, dass das Dosiseinstellglied während der Einstellung der Produktdosis verdrehfest mit dem Antriebsglied gekoppelt ist. Ein durch die rotatorisch vorgespannte Antriebsfeder, die auch als Dreh- oder Torsionsfeder bezeichnet werden kann, zwischen dem Dosiseinstellglied und dem Antriebsglied wirkendes Drehmoment, welches versucht, dass Antriebsglied relativ zu dem Dosiseinstellglied zu verdrehen, kann das Antriebsglied relativ zum dem Dosiseinstellglied nicht verdrehen, da das Dosiseinstellglied während der Einstellung der Produktdosis verdrehfest mit dem Antriebsglied gekoppelt ist. Insbesondere wird während des Einstellens der Dosis bei der Drehung des Dosiseinstellglieds in eine erste Drehrichtung oder/und in eine zweite Drehrichtung die Antriebsfeder weder gespannt noch entspannt.

Die mindestens eine Antriebsfeder, welche als Ausschüttfeder dient, kann eine Wendel- oder Schraubenfeder sein, die als Dreh- oder Torsionsfeder wirkt. Besonders bevorzugt kann die Antriebsfeder eine spiralförmig aus einem bandförmigen Material, insbesondere Metall, gewickelte Feder sein, die zum Beispiel als Spiral- oder Uhrenfeder bezeichnet werden kann. Eine rotatorisch vorgespannte Feder versucht die Teile, an denen sie sich abstützt, relativ zueinander zu verdrehen.

Die Antriebsfeder ist im Auslieferungszustand der Antriebs- und Dosiervorrichtung insbesondere mit so viel Federenergie vorgespannt, dass sie die aus dem Produktbehälter maximal oder insgesamt ausschüttbare Produktmenge in mehreren Einzelausschüttungen, d. h. insbesondere in mehreren Ausschüttungen einzelner Produktdosen, insbesondere vollständig, ausschütten kann. Die Antriebs- und Dosiervorrichtung ist so ausgestaltet, dass nach jeder Einzelausschüttung oder Ausschüttung der Produktdosis die nächste auszuschüttende Dosis neu eingestellt wird. Im Gegensatz zu Ausführungen, bei denen eine Antriebsfeder bei jeder Dosiseinstellung neu vorgespannt wird, kann durch die mit der für die Ausschüttung der maximal aus dem Produktbehälter ausschüttbaren Produktmenge erforderlichen Energie vorgespannte Feder eine einfachere Dosiseinstellung erreicht werden, da das für die Dosiseinstellung relativ zu dem Gehäuse drehbare Dosiseinstellglied dann einfacher drehbar ist, weil die Feder bei der Dosiseinstellung nicht vorgespannt werden braucht. Dies erhöht den Anwendungskomfort für den Verwender der Vorrichtung.

Das Dosiseinstellglied und das Antriebsglied können während der Einstellung der Produktdosis miteinander verdreht werden, insbesondere relativ zu dem Abtriebsglied und/oder relativ zu dem Gehäuse. Bevorzugt ist, dass eine Drehung des Dosiseinstellglieds und vorzugsweise auch das Antriebsglieds während der Einstellung der Produktdosis in eine erste Drehrichtung eine Erhöhung der auszuschüttenden Dosis bewirkt und in eine zweite, der ersten Drehrichtung entgegengesetzte Drehrichtung eine Verringerung der auszuschüttenden Produktdosis bewirkt. In Ausführungsformen, in denen eine Dosisanzeige vorgesehen ist, kann während der Drehung in die erste Drehrichtung ein Hochzählen von Skalenwerten in einer Zeigeeinrichtung wahrgenommen werden, wobei während der Drehung in die zweite Drehrichtung ein Runterzählen der Skalenwerte in der Zeigeeinrichtung wahrgenommen werden kann.

Vorzugsweise kann die Bewegung betreffend, d. h. kinematisch zwischen dem Antriebsglied und dem Abtriebsglied ein Rotationsglied angeordnet sein. Das bevorzugt hülsenförmige Rotationsglied kann das Abtriebsglied umgeben. Das Rotationsglied ist vorzugsweise axialfest und drehbar mit dem Gehäuse verbunden. Vorzugsweise greifen das Gehäuse oder ein gehäusefestes Teil und das Rotationsglied so ineinander, dass das Rotationsglied relativ zu dem Gehäuse drehbar und axialfest ist.

In einer ersten Variante kann das Rotationsglied in einem Gewindeeingriff mit einem Gewinde des Abtriebsglieds sein. Zum Beispiel kann das Abtriebsglied ein Innengewinde und das Rotationsglied ein Außengewinde aufweisen, die ineinandergreifen. Bevorzugt kann das Rotationsglied ein Innengewinde und das Abtriebsglied ein Außengewinde aufweisen, die ineinandergreifen.

Das Abtriebsglied kann mit einer gehäusefesten Führung der Antriebs- und Dosiervorrichtung in einem Eingriff sein. Zum Beispiel kann die gehäusefeste Führung ein Nocken oder ein unrunder Querschnitt sein, der in eine Nut oder in einen unrunden Querschnitt des Abtriebsglieds eingreift. Zum Beispiel kann sich die Nut oder der unrunde Querschnitt parallel zur Längsachse des länglichen Abtriebsglieds erstrecken. Die gehäusefeste Führung kann von dem Gehäuse selbst oder einem dreh- und axialfest mit dem Gehäuse verbundenen Teil gebildet werden. Dieses Teil kann permanent oder zumindest während der Produktausschüttung dreh- und axialfest mit dem Gehäuse verbunden sein. Die Nut oder der unrunde Querschnitt des Abtriebsglieds kann sich alternativ zu der Ausführung, in der sie bzw. er sich parallel zur Längsachse erstreckt, helix- oder wendelförmig um die Längsachse erstrecken, jedoch mit einer anderen Steigung als das Gewinde. Diese Varianten bewirken, dass eine Drehung des Rotationsglieds eine Bewegung des Abtriebsglieds in die distale Richtung bewirkt, insbesondere wenn das Rotationsglied in die zweite Drehrichtung relativ zu dem Gehäuse gedreht wird. Hierbei kann sich das Abtriebsglied entweder in die distale Richtung schrauben, insbesondere wenn die Nut oder der unrunde Querschnitt des Abtriebsglieds helixförmig ist, oder linear verschieben, insbesondere wenn die Nut oder der unrunde Querschnitt sich parallel zur Längsachse erstreckt.

In einer zweiten Variante kann ein gehäusefestes Innengewinde in einem Gewindeeingriff mit einem Außengewinde des Abtriebsglieds sein. Das Abtriebsglied und das Rotationsglied können so ineinandergreifen, dass das Abtriebsglied relativ zum Rotationsglied drehfest und entlang der Längsachse verschiebbar ist. Eine Drehung des Rotationsglieds bewirkt eine Schraubbewegung des Abtriebsglieds in die distale Richtung. Das Abtriebsglied kann einen unrunden Querschnitt oder eine Nut aufweisen, der bzw. die in einem Eingriff mit einem unrunden Querschnitt oder einer Abragung des Rotationsglieds ist.

Allgemein bevorzugt bewirkt eine Drehung des Antriebsglieds während der Produktausschüttung oder wenn das Betätigungsglied betätigt ist, relativ zu dem Gehäuse und/oder dem Dosiseinstellglied, dass das Abtriebsglied relativ zu dem Gehäuse in die distale Richtung bewegt wird. Insbesondere bewirkt eine Drehung des Antriebsglieds relativ zu dem Gehäuse und/oder dem Dosiseinstellglied, dass das kinematisch zwischen dem Antriebsglied und dem Abtriebsglied angeordnete Rotationsglied mit dem Antriebsglied mitgedreht wird, d. h. relativ zu dem Gehäuse und/oder dem Dosiseinstellglied bewegt wird. Die Drehung des Rotationsglieds bewirkt eine Bewegung des Abtriebsglieds in die distale Richtung. Das Abtriebsglied wird insbesondere nur während der Produktausschüttung relativ zu dem Gehäuse in die distale Richtung bewegt.

Die Antriebs- und Dosiervorrichtung kann ein Betätigungsglied zum Beispiel in der Gestalt eines Betätigungsknopfs aufweisen. Das Betätigungsglied kann insbesondere am proximalen Ende der Antriebs- und Dosiervorrichtung angeordnet sein oder das proximale Ende der Antriebs- und Dosiervorrichtung bilden. Das Betätigungsglied kann eine äußere Oberfläche der Antriebs- und Dosiervorrichtung bilden und/oder von außen zugänglich sein. Das Betätigungsglied lässt sich vorteilhaft mit dem Daumen der Hand, die das Gehäuse umgreift, betätigen, insbesondere drücken. Durch Loslassen des Betätigungsglieds kann die Betätigung beendet werden. Unter "betätigen" wird die Verschiebung des Betätigungsglieds in die Antriebs- und Dosiervorrichtung bzw. das Gehäuse oder Dosiseinstellglied, insbesondere in die distale Richtung verstanden, wodurch eine Produktausschüttung bewirkt werden kann. Das Betätigungsglied ist vorteilhalft relativ zu dem Dosiseinstellglied entlang der Längsachse verschiebbar und kann insbesondere von dem Dosiseinstellglied axial verschiebbar aufgenommen sein.

Das Betätigungsglied kann aus einer unbetätigten Position, die es während der Einstellung der auszuschüttenden Produktdosis einnimmt, in eine betätigte Position, die es während der Produktausschüttung einnimmt, verschiebbar sein. Anders ausgedrückt, ist das Betätigungsglied während der Produktausschüttung in der betätigten Position und während der Dosiseinstellung in der unbetätigten Position.

Das Betätigungsglied kann vorteilhaft gegen die Kraft einer Feder, insbesondere einer Rücksetz- oder Kupplungsfeder verschiebbar, insbesondere betätigbar sein, wobei diese Feder so auf das Betätigungsglied wirkt, dass sie gespannt wird, wenn das Betätigungsglied aus seiner unbetätigten Position in seine betätigte Position verschoben wird. Mittels der Rücksetzfeder wird das Betätigungsglied aus seiner betätigten Position in seine unbetätigte Position zurückgesetzt oder verschoben, insbesondere mit einer Bewegung in die proximale Richtung relativ zu dem Dosiseinstellglied oder/und dem Gehäuse.

Die Antriebs- und Dosiervorrichtung kann vorzugsweise eine erste Kupplung aufweisen, welche zwischen, insbesondere kinematisch zwischen dem Antriebsglied und dem Abtriebsglied angeordnet ist. Insbesondere kann die erste Kupplung zwischen dem Antriebsglied und dem Rotationsglied angeordnet sein. Die erste Kupplung ist während der Einstellung der Produktdosis oder bei unbetätigtem Betätigungsglied ausgekuppelt, wodurch dem Antriebsglied erlaubt wird, relativ zu dem Abtriebsglied verdreht zu werden. Während der Produktausschüttung oder bei betätigtem Betätigungsglied ist die erste Kupplung eingekuppelt. Hierdurch wird insbesondere erreicht, dass das Rotationsglied und das Antriebsglied verdrehfest verbunden sind. Das Rotationsglied kann somit mit dem Antriebsglied mitgedreht werden. Die erste Kupplung kann einer erste Kupplungsstruktur, welche verdrehgesichert mit dem Antriebsglied verbunden ist, insbesondere von dem Antriebsglied gebildet wird, und eine zweite Kupplungsstruktur, welche verdrehfest mit dem Rotationsglied verbunden ist, insbesondere von dem Rotationsglied gebildet wird, umfassen. Die erste Kupplungsstruktur kann eine Verzahnung sein oder aufweisen, wobei die zweite Kupplungsstruktur eine Verzahnung sein oder aufweisen kann. Die Verzahnungen der ersten Kupplungsstruktur und der zweiten Kupplungsstruktur können formschlüssig ineinandergreifen, wenn die erste Kupplung eingekuppelt ist. Zum Beispiel kann die erste Kupplungsstruktur eine Innenverzahnung sein, wobei die zweite Kupplungsstruktur eine Außenverzahnung sein kann. Alternativ kann die erste Kupplungsstruktur eine Außenverzahnung sein, wobei die zweite Kupplungsstruktur eine Innenverzahnung sein kann.

Die Antriebs- und Dosiervorrichtung weist vorzugsweise eine zweite Kupplung auf, welche zwischen dem Dosiseinstellglied und dem Antriebsglied angeordnet ist. Die zweite Kupplung ist während der Einstellung der Produktdosis oder bei unbetätigtem Betätigungsglied eingekuppelt, wodurch das Dosiseinstellglied und das Antriebsglied in Bezug zueinander verdrehgesichert sind. Während der Produktausschüttung oder bei betätigtem Betätigungsglied ist die zweite Kupplung ausgekuppelt, wodurch das Antriebsglied mittels der vorgespannten Antriebsfeder relativ zu dem Dosiseinstellglied drehbar ist.

Die zweite Kupplung kann eine dritte Kupplungsstruktur, die verdrehfest mit dem Dosiseinstellglied verbunden ist, insbesondere von dem Dosiseinstellglied gebildet wird, und eine vierte Kupplungsstruktur, die verdrehfest mit dem Antriebsglied verbunden ist, insbesondere von dem Antriebsglied gebildet wird, aufweisen. Die dritte Kupplungsstruktur kann eine Verzahnung sein oder aufweisen, wobei die vierte Kupplungsstruktur eine Verzahnung sein oder aufweisen kann. Die Verzahnungen der dritten Kupplungsstruktur und der vierten Kupplungsstruktur können formschlüssig ineinandergreifen, wenn die zweite Kupplung eingekuppelt ist. Die dritte Kupplungsstruktur kann eine Innenverzahnung sein, wobei die vierte Kupplungsstruktur eine Außenverzahnung sein kann. Alternativ kann die dritte Kupplungsstruktur eine Außenverzahnung sein, wobei die vierte Kupplungsstruktur eine Innenverzahnung sein kann.

Insbesondere können die erste und die zweite Kupplung so aufeinander abgestimmt sein, dass das Betätigungsglied zwischen der unbetätigten Position und der betätigten Position eine, zum Beispiel erste, Zwischenposition einnehmen kann, in welcher die erste Kupplung eingekuppelt ist und die zweite Kupplung eingekuppelt ist. Dies bewirkt vorteilhaft, dass die Drehung des Antriebsglieds durch die Antriebsfeder erst dann freigegeben wird, wenn sichergestellt ist, dass das Rotationsglied drehfest mit dem Antriebsglied gekoppelt ist. Hierdurch wird vorteilhaft eine Fehlfunktion der Vorrichtung vermieden, die auftreten könnte, wenn die erste Kupplung noch nicht eingekuppelt ist und die zweite Kupplung bereits ausgekuppelt ist.

Vorzugsweise kann die Antriebs- und Dosiervorrichtung eine dritte Kupplung aufweisen, welche zwischen, insbesondere kinematisch zwischen dem Dosiseinstellglied und dem Gehäuse angeordnet ist. Die dritte Kupplung ist während der Einstellung der Produktdosis oder bei unbetätigtem Betätigungsglied ausgekuppelt, wodurch das Dosiseinstellglied relativ zu dem Gehäuse verdrehbar ist. Während der Produktausschüttung oder bei betätigtem Betätigungsglied ist die dritte Kupplung eingekuppelt, wodurch das Dosiseinstellglied relativ zu dem Gehäuse drehfest ist. Allgemein ist bevorzugt, dass das Dosiseinstellglied während der Produktausschüttung relativ zu dem Gehäuse verdrehfest ist. Dies bewirkt einerseits vorteilhaft, dass während der Produktausschüttung eine Dosiseinstellung nicht möglich ist und anderseits, dass sich die Antriebsfeder mittelbar mit einem Abschnitt an dem Gehäuse abstützen kann.

Die dritte Kupplung kann zwischen dem Betätigungsglied und dem Gehäuse gebildet sein, wobei bevorzugt ist, dass das Betätigungsglied und das Dosiseinstellglied verdrehfest, insbesondere permanent verdrehfest oder zumindest während der Einstellung der Produktdosis und während der Produktausschüttung verdrehfest verbunden sind.

Die dritte Kupplung kann eine fünfte Kupplungsstruktur, die verdrehfest mit dem Gehäuse verbunden ist, insbesondere von dem Gehäuse gebildet wird, und eine sechste Kupplungsstruktur, die verdrehfest mit dem Dosiseinstellglied verbunden ist, insbesondere von dem Dosiseinstellglied oder dem Betätigungsglied gebildet wird, aufweisen. Die fünfte Kupplungsstruktur kann eine Verzahnung sein oder aufweisen, wobei die sechste Kupplungsstruktur eine Verzahnung sein oder aufweisen kann. Die Verzahnungen der fünften Kupplungsstruktur und der sechsten Kupplungsstruktur können bei eingekuppelter dritter Kupplung formschlüssig ineinandergreifen. Die fünfte Kupplungsstruktur kann eine Außenverzahnung sein, wobei die sechste Kupplungsstruktur eine Innenverzahnung sein kann. Alternativ kann die fünfte Kupplungsstruktur eine Innenverzahnung sein, wobei die sechste Kupplungsstruktur eine Außenverzahnung sein kann.

Die Antriebs- und Dosiervorrichtung kann bevorzugt eine Verdrehhemmeinrichtung, welche während der Einstellung der Produktdosis, insbesondere wenn das Betätigungsglied unbetätigt ist, verhindert, dass das Rotationsglied relativ zu dem Gehäuse, insbesondere einem drehfest oder axialverschiebbar mit dem Gehäuse verbundenen Verschiebeglied, verdrehbar ist. Die Verdrehhemmeinrichtung kann kinematisch zwischen dem Gehäuse, insbesondere dem Verschiebeglied, und dem Rotationsglied angeordnet sein. Die Verdrehhemmeinrichtung kann eine vierte Kupplung sein. Die vierte Kupplung ist während der Einstellung der Produktdosis oder bei unbetätigtem Betätigungsglied eingekuppelt, wodurch das Rotationsglied relativ zu dem Gehäuse verdrehfest ist. Während der Produktausschüttung oder bei betätigtem Betätigungsglied ist die vierte Kupplung ausgekuppelt, wodurch das Rotationsglied relativ zu dem Gehäuse verdrehbar ist.

Die vierte Kupplung kann eine siebte Kupplungsstruktur, die verdrehfest mit dem Gehäuse verbunden ist, insbesondere von dem Gehäuse oder dem Verschiebeglied gebildet wird, und eine achte Kupplungsstruktur, die verdrehfest mit dem Rotationsglied verbunden ist, insbesondere von dem Rotationsglied gebildet wird, aufweisen. Die siebte Kupplungsstruktur kann eine Verzahnung sein oder aufweisen, wobei die achte Kupplungsstruktur eine Verzahnung sein oder aufweisen kann. In einem bevorzugten Beispiel kann die Verzahnung der zweiten Kupplungsstruktur die Verzahnung der achten Kupplungsstruktur sein. Mit anderen Worten kann diese Verzahnung die zweite und die achte Kupplungsstruktur bilden. Der Vollständigkeit halber sei erwähnt, dass die zweite und achte Kupplungsstruktur alternativ separate Verzahnungen sein können.

Die Verzahnungen der siebten Kupplungsstruktur und der achten Kupplungsstruktur können bei eingekuppelter vierter Kupplung formschlüssig ineinander greifen. Die siebte Kupplungsstruktur kann eine Außenverzahnung sein, wobei die achte Kupplungsstruktur eine Innenverzahnung sein kann. Alternativ kann die siebte Kupplungsstruktur eine Innenverzahnung sein, wobei die achte Kupplungsstruktur eine Außenverzahnung sein kann.

Alternativ kann die Verdrehhemmeinrichtung ein von dem Rotationsglied gebildetes Rastmittel, welches vorzugsweise elastisch nachgiebig von dem Rotationsglied gebildet wird, und eine Verzahnung, vorzugsweise Innenverzahnung sein, die verdrehfest in Bezug auf das Gehäuse ist, insbesondere von dem Gehäuse oder dem Verschiebeglied gebildet wird. Das Rastmittel kann in die Verzahnung eingreifen, wobei das Rotationsglied nur nach Überwindung eines gewissen Grenzdrehmoments relativ zu dem Gehäuse oder dem Verschiebeglied verdreht werden kann. Durch Abstimmung des Rastmittels und der Innenverzahnung kann das Grenzdrehmoment so ausgelegt werden, das es ohne weiteres von einem Drehmoment, welches durch die rotatorisch vorgespannte Antriebsfeder während der Produktausschüttung bereitgestellt wird, überwunden werden kann.

In einer bevorzugten Ausführungsform kann sowohl die vierte Kupplung als auch das in die Verzahnung eingreifende Rastmittel vorhanden sein, wobei der Eingriff des Rastmittels dann nicht als Verdrehhemmeinrichtung dient sondern lediglich der Erzeugung eines akustischen und/oder taktilen Signals während der Produktausschüttung.

Die hierin beschriebenen Kupplungen bzw. Kupplungsstrukturen können mit Bewegungen entlang der Längsachse der Antriebs- und Dosiervorrichtung formschlüssig ineinander geschoben bzw. eingekuppelt werden. Vorzugsweise ist das Antriebsglied durch Betätigen und Loslassen des Betätigungsglieds relativ zum Gehäuse entlang der Längsachse hin und her verschiebbar, wodurch die erste Kupplungsstruktur und die vierte Kupplungsstruktur und - sofern vorhanden - die siebte Kupplungsstruktur entlang der Längsachse verschoben werden. Durch Betätigen des Betätigungsglieds wird insbesondere auch die sechste Kupplungsstruktur relativ zum Gehäuse entlang der Längsachse in die distale Richtung verschoben.

Vorzugsweise kann das Betätigungsglied eine weitere, insbesondere eine zweite Zwischenposition einnehmen, in welcher die dritte Kupplung und die zweite Kupplung eingekuppelt sind. Hierdurch wird vorteilhaft sichergestellt, dass die Antriebsfeder kinematisch zwischen das Gehäuse und das Antriebsglied geschaltet wird, bevor die Drehung des Antriebsglieds relativ zu dem Gehäuse freigegeben wird.

Insbesondere kann die optional vorhandene vierte Kupplung, die letzte der Kupplungen sein, die gekuppelt wird, wenn das Betätigungsglied aus seiner unbetätigten Position in seine betätigte Position verschoben wird. Hierdurch wird bewirkt, dass das Auskuppeln der vierten Kupplung das Drehmoment der Antriebsfeder auf das Rotationsglied schaltet, wodurch das Rotationsglied gedreht wird.

Insbesondere ist es allgemein bevorzugt, dass die Antriebsfeder während der Dosiseinstellung zwischen das Dosiseinstellglied und das Antriebsglied geschaltet ist, und während der Produktausschüttung zwischen das Gehäuse und das Antriebsglied geschaltet ist. Insbesondere die dritte Kupplung übernimmt die Funktion des Schalters.

Die Antriebsfeder kann einen ersten Abschnitt mit dem sie sich während der Einstellung der auszuschüttenden Produktdosis in Bezug auf das Dosiseinstellglied oder während der Produktausschüttung in Bezug auf das Gehäuse verdrehfest abstützt, einen zweiten Abschnitt mit dem sie sich in Bezug auf das Antriebsglied verdrehfest abstützt, und einen dritten Abschnitt, der zwischen dem ersten Abschnitt und dem zweiten Abschnitt angeordnet ist und der sich bei einer Änderung der Federspannung der Antriebsfeder elastisch verformt, aufweisen. Vorzugsweise werden der erste Abschnitt und der zweite Abschnitt relativ zu dem Gehäuse verdreht, wenn das Dosiseinstellglied relativ zu dem Gehäuse verdreht wird. Insbesondere können der erste Abschnitt und der zweite Abschnitt in Bezug zueinander stillstehen, wenn das Dosiseinstellglied für die Dosiseinstellung gedreht wird. Hierdurch wird bewirkt, dass sich die Federspannung während der Dosiseinstellung nicht ändert. Der erste Abschnitt kann an dem Betätigungsglied befestigt sein oder daran anliegen. Der zweite Abschnitt kann an dem Antriebsglied befestigt sein oder daran anliegen.

Vorzugsweise umgibt das Betätigungsglied die Antriebsfeder und/oder einen zum Beispiel stiftförmigen Abschnitt des Antriebsglieds. Die Antriebsfeder kann den Abschnitt des Antriebsglieds umgeben. Besonders bevorzugt kann die Antriebsfeder in dem Betätigungsglied aufgenommen sein und mit dem ersten Abschnitt an dem Betätigungsglied befestigt sein und/oder anliegen und hierdurch mittelbar an dem Dosiseinstellglied und/oder dem Gehäuse abgestützt sein, insbesondere je nach Schaltzustand der dritten Kupplung.

Zwischen dem Antriebsglied und dem Betätigungsglied kann in bevorzugten Ausführungen eine Einrichtung angeordnet sein, welche insbesondere die Reibung zwischen dem Betätigungsglied und dem Antriebsglied verringert, wenn sich das Antriebsglied während der Produktausschüttung relativ zu dem Betätigungsglied dreht. Die Einrichtung kann zum Beispiel in der Gestalt sein, dass sich das proximale Ende des Antriebsglieds, insbesondere des Abschnitts, an dem die Ausschüttfeder befestigt ist, zu einer Kontaktfläche des Betätigungsglieds hin verjüngt, wobei zwischen dem proximalen Ende des Antriebsglieds und der Kontaktfläche ein Spalt besteht, wenn das Betätigungsglied in seiner unbetätigten Position ist und das proximale Ende des Antriebsglieds an der Kontaktfläche anliegt, wenn das Betätigungsglied in seiner betätigten Position ist. Hierdurch kann einerseits bewirkt werden, dass das Betätigungsglied das Antriebsglied bei der Verschiebung des Betätigungsglieds in seine betätigte Position mitnimmt, wodurch die erste und die zweite Kupplung ein- bzw. ausgekuppelt werden. Durch den Kontakt des Antriebsglieds mit dem Betätigungsglied entsteht Reibung zwischen dem Antriebsglied und Betätigungsglied, wenn sich das Antriebsglied relativ zu dem Betätigungsglied dreht. Durch die genannte Anordnung kann diese Reibung verringert werden. Das proximale Ende kann zum Beispiel kegelförmig, kegelstumpfförmig oder kugelförmig ausgebildet sein. Alternativ kann die Kontaktfläche von einem zwischen dem Antriebsglied und dem Betätigungsglied angeordneten reibungsvermindernden Einsatz gebildet werden. Der reibungsmindernde Einsatz kann zum Beispiel ein Einsatz aus einem reibungsvermindernden Kunststoff, wie zum Bespiel Teflon, POM oder dergleichen sein. Alternativ kann ein Wälzlager, insbesondere ein Axialwälzlager oder Axialkugellager zwischen dem Antriebsglied und dem Betätigungsglied angeordnet sein.

In bevorzugten Ausführungen kann das Betätigungsglied ein Eingriffsglied aufweisen, welches das Antriebsglied daran hindert, relativ zu dem Betätigungsglied, beispielsweise durch die Rücksetzfeder in die distale Richtung verschoben zu werden, wenn das Betätigungsglied unbetätigt ist. Zum Beispiel kann das Eingriffsglied in das Antriebsglied so eingreifen, dass diese Bewegung verhindert wird. Das Antriebsglied kann zum Beispiel einen ringförmig umlaufenden Kragen aufweisen, den das Betätigungsglied, insbesondere das Eingriffsglied umgreift.

Allgemein bevorzugt kann die Rücksetzfeder zwischen dem Dosiseinstellglied und dem Betätigungsglied angeordnet sein, insbesondere sich mit einem Ende, insbesondere dem distalen Ende an dem Dosiseinstellglied und mit dem anderen Ende, insbesondere an dem proximalen Ende an dem Betätigungsglied abstützen. Die Rücksetzfeder kann insbesondere eine Wendelfeder sein, die als Druckfeder wirkt.

Beispielsweise kann die Antriebsfeder in dem zum Beispiel stiftförmigen Abschnitt des Antriebsglieds befestigt sein, der zwischen dem distalen Ende des Antriebsglieds und dem ringförmig umlaufenden Kragen angeordnet ist.

Das Betätigungsglied kann zum Beispiel mehrteilig, wie zum Beispiel zweiteilig sein, wobei es ein Verbindungsglied aufweisen kann, welches zum Beispiel topfförmig ausgestaltet ist und in welches die Antriebsfeder eingesetzt ist, wobei das Verbindungsglied an seinem distalen Ende mittels einer Abdeckkappe des Betätigungsglieds verschlossen ist. Die Abdeckkappe kann die Kontaktfläche für zum Bespiel den Daumen des Verwenders der Antriebs- und Dosiervorrichtung bilden. Das Verbindungsglied kann zum Beispiel die sechste Kupplungsstruktur und/oder das Eingriffsglied, welches den Kragen des Antriebsglieds umgreift, aufweisen.

Besonders bevorzugte Ausführungen der Antriebs- und Dosiervorrichtung umfassen ein Dosisanzeigeelement, welches an seinem Außenumfang eine Dosisskala aufweist und verdrehfest mit dem Antriebsglied verbunden ist, insbesondere permanent, d. h. während der Produktausschüttung und während der Dosiseinstellung. Das Dosisanzeigeelement macht somit die Drehbewegungen des Antriebsglieds mit. Zum Beispiel können das Antriebsglied und das Dosisanzeigeelement so ineinandergreifen, dass das Antriebsglied und das Dosisanzeigeelement relativ zueinander verdrehfest und entlang der Längsachse verschiebbar sind. Das Gehäuse weist eine Zeigeeinrichtung auf, insbesondere ein Fenster. Durch die Zeigeeinrichtung kann ein Skalenwert der Dosisskala, welcher mit der eingestellten Dosis korrespondiert, abgelesen werden. Das Dosisanzeigeelement kann zum Beispiel im Querschnitt ringförmig sein. Das Dosisanzeigeelement kann zum Beispiel eine Dosisanzeigetrommel oder Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Dosiswerten, die aneinandergereiht angeordnet sind und die Dosisskala ergeben. Vorzugsweise handelt es sich um Zahlenwerte, welche die gewünschte Produktdosis in internationalen Einheiten (IU) oder in Milligramm angeben.

Alternativ kann die Dosisskala ohne Steigung über den Umfang des Dosisanzeigeelements, wie zum Beispiel des Dosisanzeigerings, angeordnet sein, wobei sich die Skalenwerte dann nach einer Umdrehung des Dosisanzeigeelements wiederholen. Bei einer Dosisskala mit Steigung, d. h. einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte dargestellt werden können.

Das Dosisanzeigeelement ist zur Einstellung der zu verabreichenden Dosis relativ zu der Zeigeeinrichtung und insbesondere um eine Drehachse, die vorzugsweise der Längsachse des Antriebs- und Dosiermoduls und/oder des Dosisanzeigeelements entspricht, drehbar. Hierbei kann es sich um eine reine Drehbewegung, d. h. eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Dosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegungen und die Dosisskala vorteilhafterweise die gleiche Steigung aufweisen. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann zum Bespiel ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der zum Beispiel zusätzlich zu dem Fenster den Wert der Dosisskala markiert, welcher der eingestellten Dosis entspricht. Dies ist dann zum Beispiel von Vorteil, wenn in dem Fenster noch ein anderer Wert zumindest teilweise erscheint, um eine eindeutige Dosisauswahl sicherzustellen. Der Zeiger kann zum Beispiel ein Vorsprung oder ein Aufdruck oder eine Kerbe oder dergleichen sein.

Insbesondere kann das Dosisanzeigeelement zumindest während der Dosiseinstellung verdrehfest, aber zum Beispiel axial verschiebbar mit dem Dosiseinstellelement verbunden oder gekoppelt sein. Für die intuitive Bedienung ist es vorteilhaft, wenn das Dosisanzeigeelement bei Drehung des Dosierglieds um einen Drehwinkel ebenfalls um diesen Drehwinkel verdreht wird.

Das Dosisanzeigeelement kann ein Gewinde aufweisen, welches in einem Eingriff mit dem Gehäuse oder einem gehäusefesten Elements ist oder alternativ mit einem Verschiebeglied, welches auch als Lagerelement bezeichnet werden kann, in einem Gewindeeingriff ist, wobei das Verschiebeglied drehfest und entlang der Längsachse verschiebbar in Bezug auf das Gehäuse ist. Der Gewindeeingriff bewirkt vorteilhaft die Dreh- oder Schraubbewegung des Dosisanzeigeelements relativ zu der Zeigeeinrichtung. Zum Beispiel kann der Eingriff zwischen Dosisanzeigeelement und Verschiebeglied oder Gehäuse ein Gewindeeingriff sein. Insbesondere kann das Verschiebeglied ein Außengewinde und das Dosisanzeigeelement ein Innengewinde aufweisen, wobei diese Gewinde ineinandergreifen und dadurch bewirken, dass das Dosisanzeigeelement relativ zu dem Verschiebeglied schraubbar ist.

Insbesondere ist das Dosisanzeigeelement zwischen einer Maximaldosisposition und einer Nulldosisposition hin und her drehbar oder schraubbar. In der Nulldosisposition kann vorteilhafte Dosis oder Ziffer "null" in der Zeigeeinrichtung ablesbar sein. In der Maximaldosisposition kann vorteilhaft die maximal mit dem Antriebs- und Dosiermodul ausschüttbare Produktdosis ablesbar sein.

In der Nulldosisposition kann das Dosisanzeigeelement gegen die Drehung in eine Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung, insbesondere in die erste Drehrichtung, bewegt werden, die eine Erhöhung der Dosis bewirkt. In der Maximaldosisposition ist das Dosisanzeigeelement vorzugsweise gegen die Drehung in eine Drehrichtung, nämlich in die Drehrichtung, die die Einstellung einer Dosis über die maximal einstellbare Dosis hinausbewirken würde, blockiert. Das Dosisanzeigeelement kann in der Maximaldosisposition insbesondere nur in die Drehrichtung, insbesondere die zweite Drehrichtung, gedreht werden, die eine Verringerung der Produktdosis bewirkt.

Das Dosisanzeigeelement kann zum Beispiel einen Anschlag aufweisen, der in der Nulldosisposition an einen Gegenanschlag anschlägt und somit die Drehung in eine Drehrichtung verhindert. Der gleiche oder ein zusätzlicher Anschlag des Dosisanzeigeelements kann die Drehung des Dosisanzeigeelements über die Maximaldosis hinaus verhindern. Insbesondere kann hierfür ein weiterer Gegenanschlag nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung.

Der Nulldosisgegenanschlag kann zum Bespiel von dem Gehäuse, insbesondere einem gehäusefesten Element, oder dem Verschiebeglied gebildet werden. Der Maximaldosisgegenanschlag kann zum Bespiel von dem Gehäuse, insbesondere einem gehäusefesten Element, oder dem Verschiebeglied gebildet werden.

Insbesondere durch Betätigen des Betätigungsglieds kann das Verschiebeglied zusammen mit dem Dosisanzeigeelement relativ zu dem Gehäuse entlang der Drehachse, insbesondere in distale Richtung verschiebbar sein. Alternativ kann das Dosisanzeigeelement ein Gewinde aufweisen, welches in dem Eingriff mit dem Gehäuse oder einem gehäusefesten Element ist. Dadurch lasst sich das Dosisanzeigeelement zwar relativ zu dem Gehäuse hin und her schrauben aber nicht unabhängig von der Schraubbewegung mit einer insbesondere reinen Axialbewegung verschieben.

Vorzugsweise ist das Betätigungsglied so mit dem Verschiebeglied gekoppelt, dass eine Verschiebung des Betätigungsglieds relativ zu dem Gehäuse und/oder dem Dosierglied eine Verschiebung des Verschiebeglieds relativ zu dem Gehäuse und/oder dem Dosierglied, insbesondere entlang der Längsachse des Antriebs- und Dosiermoduls, bewirkt. Allgemein bevorzugt können das Verschiebeglied und das Antriebsglied so miteinander verbunden sein, insbesondere ineinandergreifen, dass das Antriebsglied in Bezug auf das Verschiebeglied drehbar und axialfest ist.

Dadurch, dass das Dosisanzeigeelement in einem Eingriff mit dem Verschiebeglied ist und sich das Verschiebeglied relativ zu dem Gehäuse und entlang der Drehachse verschieben lässt, lässt sich auch das Dosisanzeigeelement relativ zu dem Gehäuse und entlang der Drehachse unabhängig von der Dreh- oder Schraubbewegung, welche das Dosisanzeigeelement bei der Dosiseinstellung ausführt, verschieben. Die Antriebs- und Dosiervorrichtung lässt sich im Grunde aber auch vorteilhaft mit dem alternativen Dosisanzeigeelement kombinieren, das in dem Gewindeeingriff mit dem Gehäuse oder einem gehäusefesten Element ist. In dieser Alternative kann das Verschiebeglied vorzugsweise drehfest und axial verschiebbar in Bezug auf das Gehäuse sein.

Vorteilhaft kann an der Zeigeeinrichtung und/oder an dem Dosisanzeigeelement abgelesen werden, dass das Verschiebeglied zusammen mit dem Dosisanzeigeelement verschoben wurde. Hierdurch lässt sich durch den Verwender kontrollieren, in welchem Betriebszustand sich die Antriebs- und Dosiervorrichtung befindet, d. h. ob die Antriebs- und Dosiervorrichtung, insbesondere das Betätigungsglied, für eine Ausschüttung betätigt oder unbetätigt ist.

In einer bevorzugten Variante kann das Betätigungsglied oder/und das Verschiebeglied zusammen mit dem Dosisanzeigeelement relativ zu der Zeigeeinrichtung, dem Gehäuse und entlang der Drehachse verschiebbar sein. Im Bereich der Zeigeeinrichtung, insbesondere in dem Fenster der Zeigeeinrichtung, kann eine von der Dosisskala verschiedene Markierung erscheinen, wenn das Verschiebeglied verschoben ist. Die Markierung ist vorzugsweise an dem Dosisanzeigeelement angeordnet. Wenn das Verschiebeglied unverschoben ist, insbesondere die Antriebs- und Dosiervorrichtung für die Produktausschüttung unbetätigt ist, kann die Markierung außerhalb der Zeigeeinrichtung angeordnet sein, wie z. B. von einem Gehäuse oder einem anderen Element verdeckt sein. Wird das Verschiebeglied verschoben, insbesondere die Antriebs- und Dosiervorrichtung für die Produktausschüttung betätigt, kann die Markierung aus dem abgedeckten Bereich hervortreten, so dass sie insbesondere an oder in der Zeigeeinrichtung erscheint oder ablesbar ist. Wird die Betätigung der Antriebs- und Dosiervorrichtung unterbrochen oder beendet, kann das Verschiebeglied in die Ursprungsposition zurückkehren, wodurch die Markierung vorzugsweise aus dem Bereich der Zeigeeinrichtung entfernt und insbesondere verdeckt wird.

In einer alternativen Variante kann das Betätigungsglied oder/und das Verschiebeglied zusammen mit dem Dosisanzeigeelement und der Zeigeeinrichtung relativ zu dem Gehäuse und entlang der Drehachse verschiebbar sein. Die Zeigeeinrichtung kann z. B. eine Blende sein oder zumindest die Funktion einer Blende erfüllen. Z. B. kann die Zeigeeinrichtung zumindest axial fest, vorzugsweise auch drehfest mit dem Verschiebeglied verbunden sein. Im Grunde kann das Verschiebeglied die Zeigeeinrichtung bilden. Selbstverständlich ist es aber auch möglich, dass die Zeigeeinrichtung ein von dem Verschiebeglied separates Teil ist. Die Zeigeeinrichtung kann z. B. hülsenförmig sein.

In dieser Variante kann die Verschiebung des Verschiebeglieds bewirken, dass im Bereich der Zeigeeinrichtung eine von der Dosisskala verschiedene Markierung erscheint, die an oder auf der Zeigeeinrichtung angeordnet oder gebildet ist. Beispielsweise kann die Zeigeeinrichtung innerhalb des Gehäuses angeordnet sein. Die Markierung der Zeigeeinrichtung kann in unbetätigtem Zustand der Antriebs- und Dosiervorrichtung von dem Gehäuse oder einem anderem Element verdeckt sein. Wird die Antriebs- und Dosiervorrichtung, insbesondere das Betätigungsglied, betätigt, so dass das Dosisanzeigeelement zusammen mit der Zeigeeinrichtung verschoben wird, kann die Markierung aus seiner Abdeckung hervortreten, so dass die Markierung erblickbar oder ablesbar ist. Wird die Betätigung unterbrochen oder beendet, kann das Dosisanzeigeelement zusammen mit der Zeigeeinrichtung und dem Verschiebeglied in seine Ausgangsposition zurückverschoben werden, so dass die Markierung wieder unter der Abdeckung angeordnet ist.

Allgemein bevorzugt kann bei der Betätigung der Antriebs- und Dosiervorrichtung für eine Produktausschüttung eine Feder, insbesondere eine Kupplungs- oder Rückstellfeder gespannt werden. Mit anderen Worten ausgedrückt kann das Verschiebeglied bei der Betätigung gegen die Kraft einer insbesondere solchen Feder verschoben werden, insbesondere aus einer unbetätigten Position in eine betätigte Position. Die Feder kann z. B. eine Schrauben- oder Wendelfeder sein, die als Druckfeder wirkt. Diese Feder bewirkt ferner, dass beim Unterbrechen oder Beenden der Betätigung das Verschiebeglied in seine Ausgangsposition oder unbetätigte Position zurückgesetzt wird. Insbesondere wird das Verschiebeglied bei der Betätigung in die distale Richtung verschoben. Mittels der Feder wird das Verschiebeglied in die proximale Richtung zurück verschoben, wenn die Betätigung unterbrochen oder beendet wird.

Die Erfindung wurde anhand mehrerer bevorzugter Ausführungen beschrieben. Im Folgenden werden besonders bevorzugte Ausführungen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1: eine Explosionsdarstellung der Einzelteile einer erfindungsgemäßen Antriebs- und Dosiervorrichtung nach einer ersten Ausführungsform,
- Figur 2: die Explosionsdarstellung aus Figur 1, wobei die Einzelteile im Schnitt dargestellt sind,
- Figur 3: verschiedene Ansichten der aus den wesentlichen Teilen aus Figur 1 zusammengesetzten Antriebs- und Dosiervorrichtung in einem Auslieferungszustand,
- Figur 4: die Antriebs- und Dosiervorrichtung aus Figur 3 in einem Zustand, in der eine maximal ausschüttbare Dosis eingestellt ist,
- Figur 5: die Antriebs- und Dosiervorrichtung aus Figur 3 in einem betätigten Zustand nach der Produktausschüttung,
- Figur 6: die Antriebs- und Dosiervorrichtung aus Figur 3, bei der das in dem Produktbehälter enthaltene Produkt vollständig ausgeschüttet wurde,
- Figur 7: eine Explosionsdarstellung der Einzelteile einer erfindungsgemäßen Antriebs- und Dosiervorrichtung nach einer zweiten Ausführungsform,
- Figur 8: die Explosionsdarstellung aus Figur 7, wobei die Einzelteile im Schnitt dargestellt sind,
- Figur 9: verschiedene Ansichten der aus den wesentlichen Teilen aus Figur 7 zusammengesetzten Antriebs- und Dosiervorrichtung in einem Auslieferungszustand und
- Figur 10: die Antriebs- und Dosiervorrichtung aus Figur 9 in einem betätigten Zustand bei maximal eingestellter Dosis.

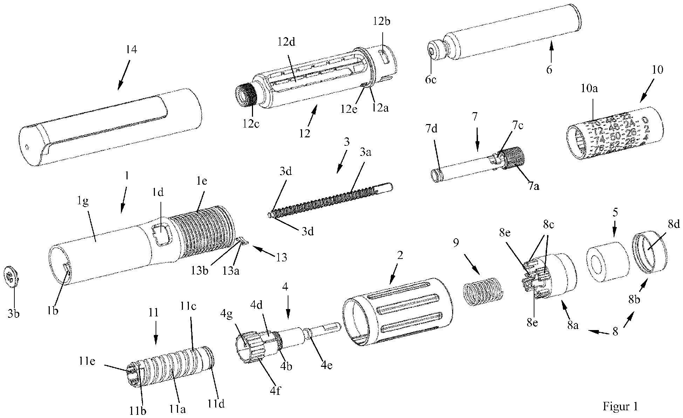

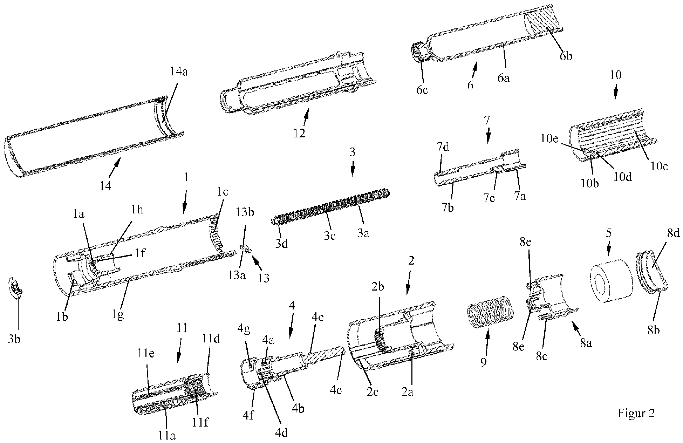

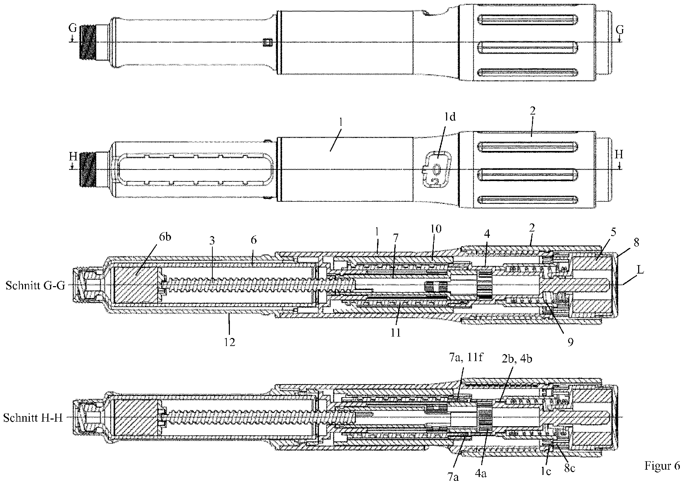

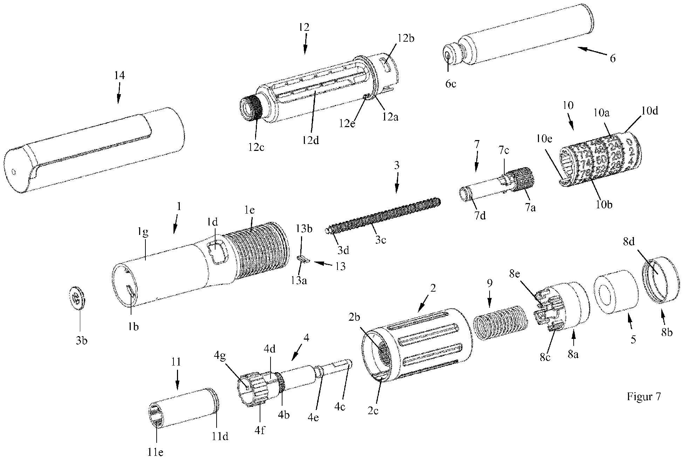

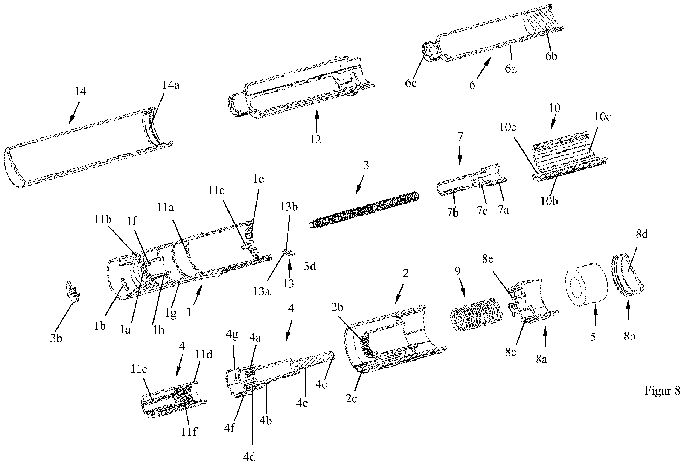

Die erste Ausführungsform aus den Figuren 1 bis 6 und die zweite Ausführungsform aus den Figuren 7 bis 10 unterscheiden sich im Wesentlichen in der Ausgestaltung eines Dosisanzeigeelements 10 und eines Verschiebeglieds 11. In der ersten Ausführungsform weist das Dosisanzeigeelement 10 ein Innengewinde 10b auf, welches in ein Außengewinde 11a des Verschiebeglieds 11 (Figur 1) eingreift. Die zweite Ausführungsform weist ein Dosisanzeigeelement 10 auf, welches ein Außengewinde 10b aufweist, welches in ein Innengewinde 11a des Gehäuses 1 (Figur 8) eingreift.

Sofern nichts anderes angegeben wird, bezieht sich die folgende Beschreibung auf die erste Ausführungsform und die zweite Ausführungsform.

Die Antriebs- und Dosiervorrichtung bildet eine Injektionsvorrichtung oder ist zumindest ein Teil einer solchen Injektionsvorrichtung. Die Antriebs- und Dosiervorrichtung weist ein hülsenförmiges Gehäuse 1 auf, welches eine Außenhülse 1g und eine damit verbundene und konzentrisch dazu angeordnete Innenhülse 1h aufweist. Die Innenhülse 1h und die Außenhülse 1g sind über einen ringförmigen Steg fest verbunden. Das Gehäuse 1, insbesondere die Innenhülse 1h weist einen Innengewinde 1a auf, welches in ein Außengewinde 3c der Gewindestange 3a eingreift, so dass die Gewindestange 3a und somit das Abtriebsglied 3, relativ zu dem Gehäuse 1 und entlang der Längsachse L in die distale Richtung geschraubt werden kann. Das Abtriebsglied 3 weist die Gewindestange 3a und einen tellerförmigen Flansch 3b auf, der frei drehbar am distalen Ende der Gewindestange 3a befestigt, insbesondere verschnappt ist. Die Gewindestange 3a weist mindestens eine Führungsnut 3d auf, welche das Außengewinde 3c überlagert und sich parallel zur Längsachse L erstreckt. Ein hülsenförmiges Rotationsglied 7 weist an seinem Innenumfang mindestens ein stegförmiges Eingriffsglied 7b auf, welches in die Führungsnut 3d eingreift, wodurch das Rotationsglied 7 und das Abtriebsglied 3 relativ zueinander verdrehfest und axial verschiebbar sind. Das Rotationsglied 7 weist an seinem Außenumfang eine ringförmige Nut 7d auf, in welche eine am Innenumfang des Gehäuses 1, insbesondere der Innenhülse 1h gebildete Abragung 1f eingreift, wodurch das Rotationsglied 7 relativ zu dem Gehäuse 1 verdrehbar und axialfest ist. Eine Drehung des Rotationsglieds 7 bewirkt eine Drehung des Abtriebsglieds 3, wodurch das Abtriebsglied 3 aufgrund des Gewindeeingriffs mit dem Gehäuse 1 entlang der Längsachse L bewegbar ist. Beispielsweise wird das Abtriebsglied 3 in die distale Richtung bewegt, wenn das Rotationsglied 7 relativ zu dem Gehäuse 1 in eine zweite Drehrichtung um die Längsachse L gedreht wird.

Das Rotationsglied 7 weist eine zweite Kupplungsstruktur 7a in Gestalt einer Außenverzahnung auf.

Das Gehäuse 1, insbesondere die Innenhülse 1h weist an ihrem Außenumfang eine Eingriffsstruktur auf, wobei diese Eingriffsstruktur und eine am Innenumfang des Verschiebeglieds 11 gebildete Nut oder rippenförmige Längsführung 11e so ineinandergreifen, dass das Verschiebeglied 11 relativ zu dem Gehäuse 1 um die Längsachse L verdrehgesichert und entlang der Längsachse L verschiebbar ist. Das Verschiebglied 11 weist an seinem Innenumfang eine Innenverzahnung 11f auf, in welche ein Rastmittel 7c, welches federnd an dem Rotationsglied 7 gebildet ist, eingreift. Das Rastmittel 7c weist einen Rastarm auf, an dessen Außenseite eine Eingriffsnocke angeordnet ist, welche in die Innenverzahnung 11f eingreift. Bei der Drehung des Rotationsglieds 7 relativ zu dem Verschiebeglied 11 gleitet das Rastmittel 7c über die Innenverzahnung 11f, wodurch zum Beispiel mittels eines akustischen und/oder taktilen Signals die Produktausschüttung signalisiert werden kann.

Der Eingriff des Rastmittels 7c in die Innenverzahnung 11f kann in einer Variante derart sein, dass das Rotationsglied 7 nur nach Überwindung eines gewissen Grenzdrehmoments relativ zu dem Gehäuse 1 oder dem Verschiebeglied 11 gedreht werden kann. Hierdurch verhindert der Eingriff des Rastmittels 7c in die Innenverzahnung 11f, dass sich das Abtriebsglied 3 unbeabsichtigt relativ zu dem Gehäuse 1 verdreht, wie zum Beispiel durch Vibrationen beim Transport der Antriebs- und Dosiervorrichtung. Durch Abstimmung des Rastmittels 7c und der Innenverzahnung 11f ist das Grenzdrehmoment so ausgelegt, dass es ohne weiteres von einem Drehmoment, welches durch eine rotatorische vorgespannte Antriebsfeder 5 während der Produktausschüttung bereitgestellt wird, überwunden werden kann.

In den Figuren wird jedoch eine andere, bevorzugte Variante gezeigt, in der das Rastmittel 7c die Innenverzahnung 11f lediglich zur Erzeugung eines akustischen und/oder taktilen Signals während der Produktausschüttung dienen. Die Innenverzahnung 11f bildet eine siebte Kupplungsstruktur, welche in die Außenverzahnung der zweiten Kupplungsstruktur 7a eingreift. Die zweite Kupplungsstruktur 7a und die siebte Kupplungsstruktur 11f bilden eine vierte Kupplung 7a, 11f. Alternativ kann das Rotationsglied 7 eine achte Kupplungsstruktur in der Gestalt einer Verzahnung, insbesondere Außenverzahnung aufweisen, die zum Beispiel separat von der zweiten Kupplungsstruktur 7a ist. In diesem Fall können die siebte Kupplungsstruktur 11f und die achte Kupplungsstruktur die vierte Kupplung bilden.

Die vierte Kupplung 7a, 11f ist eingekuppelt, wenn das Betätigungsglied 8 unbetätigt ist, und ausgekuppelt, wenn das Betätigungsglied 8 betätigt ist. Wenn die vierte Kupplung 7a, 11f eingekuppelt ist, greifen die siebte Kupplungsstruktur 11f und die zweite Kupplungsstruktur 7a oder die achte Kupplungsstruktur verdrehfest ineinander.

Das Verschiebeglied 11 weist an seinem proximalen Ende eine Ringnut 11d auf, in welche eine Abragung 4g am Innenumfang eines Antriebsglieds 4 eingreift, wodurch das Antriebsglied 4 relativ zu dem Verschiebeglied 11 verdrehbar und axialfest ist. Eine Verschiebung des Antriebsglieds 4 entlang der Längsachse L bewirkt somit auch eine Verschiebung des Verschiebeglieds 11 entlang der Längsachse L. Das Antriebsglied 4 weist eine erste Kupplungsstruktur 4a in Gestalt einer Innenverzahnung auf, welche mit der zweiten Kupplungsstruktur 7a eine erste Kupplung 4a, 7a bildet. Das Antriebsglied 4 ist aus einer ausgekuppelten Position, in welcher die erste Kupplungsstruktur 4a und die zweite Kupplungsstruktur 7a nicht ineinandergreifen, in eine eingekuppelte Position entlang der Längsachse L verschiebbar, in welcher die erste Kupplungsstruktur 4a und die zweite Kupplungsstruktur 7a formschlüssig ineinandergreifen. Das Antriebsglied 4 ist relativ zu dem Rotationsglied 7 um die Längsachse L drehbar, wenn die erste Kupplung 4a, 7a ausgekuppelt ist, und relativ zu dem Rotationsglied 7 um die Längsachse L verdrehfest, wenn die erste Kupplung 4a, 7a eingekuppelt ist.

Das Antriebsglied 4 weist eine Raststruktur 4d auf, welche federnd an dem Antriebsglied 4 gebildet ist. Die Raststruktur 4d weist mindestens einen Zahn auf, welcher in einem Eingriff mit einer Verzahnung, insbesondere der zweiten Kupplungsstruktur 7a des Rotationsglieds 7 ist, wenn die erste Kupplung 4a, 7a ausgekuppelt ist. Die Raststruktur 4d rastet über die Verzahnung, insbesondere die zweite Kupplungsstruktur 7a des Rotationsglieds 7, wenn das Antriebsglied 4 relativ zu dem Rotationsglied 7 während der Einstellung einer auszuschüttenden Produktdosis in eine erste Drehrichtung oder/und eine zweite Drehrichtung verdreht wird. Hierdurch werden einerseits Klickgeräusche erzeugt, welche dem Verwender das Einstellen der Dosis taktil und/oder akustisch signalisieren und andererseits diskrete Winkelpositionen für das Antriebsglied 4 in Bezug auf das Rotationsglied 7 vorgegeben.

In der Variante ohne vierter Kupplung 7a, 11f kann der Eingriff der Raststruktur 4d in das Rotationsglied 7 in Bezug auf den Eingriff des Rastmittels 7c in die Innenverzahnung 11f so ausgelegt sein, dass bei einer Drehung des Antriebsglieds 4 relativ zu dem Rotationsglied 7 das hierdurch auf das Rotationsglied 7 ausgeübte Drehmoment geringer ist als das Grenzdrehmoment, welches für eine Drehung des Rotationsglieds 7 in Bezug auf das Verschiebeglied 11 notwendig ist.

Das Antriebsglied 4 weist eine vierte Kupplungsstruktur 4b auf, die als Außenverzahnung ausgestaltet ist. Die vierte Kupplungsstruktur 4b bildet mit einer dritten Kupplungsstruktur 2b, die als Innenverzahnung ausgestaltet ist, eine zweite Kupplung 2b, 4b. An dem Gehäuse 1 ist ein hülsenförmiges Dosiseinstellglied 2 befestigt, wobei das Dosiseinstellglied 2 relativ zu dem Gehäuse 1 drehbar und axialfest ist. Das Dosiseinstellglied 2 weist eine Außenhülse und eine Innenhülse auf, die über einen Steg fest miteinander verbunden sind. Das Dosiseinstellglied 2, insbesondere die Außenhülse weist an ihrem Innenumfang eine Abragung 2a auf, welche in eine Ringnut des Gehäuses 1, insbesondere der Außenhülse 1g eingreift, so dass das Dosiseinstellglied 2 relativ zu dem Gehäuse 1 drehbar und axialfest ist.

Das Dosiseinstellglied 2, insbesondere deren Innenhülse, bildet die dritte Kupplungsstruktur 2b. Die dritte Kupplungsstruktur 2b greift formschlüssig in die vierte Kupplungsstruktur 4b, wenn die zweite Kupplung 2b, 4b eingekuppelt ist, wodurch das Dosiseinstellglied 2 um die Längsachse L drehfest mit dem Antriebsglied 4 verbunden ist. Das Antriebsglied 4 macht somit Drehbewegungen des Dosiseinstellglieds 2 mit. Wenn die zweite Kupplung 2b, 4b ausgekuppelt ist, greifen die dritte Kupplungsstruktur 2b und die vierte Kupplungsstruktur 4b nicht ineinander, so dass das Dosiseinstellglied 2 und das Antriebsglied 4 relativ zueinander verdrehbar sind.

Für die Einstellung einer auszuschüttenden Produktdosis wird das Dosiseinstellglied 2 relativ zu dem Gehäuse 1 zur Erhöhung der Dosis in eine erste Drehrichtung und zur Verringerung der Korrektur der Dosis in eine zweite Drehrichtung gedreht. Während der Dosiseinstellung ist die zweite Kupplung 2b, 4b eingekuppelt, wodurch das Antriebsglied 4 die Drehbewegungen des Dosiseinstellglieds 2 mitmacht.

Das Dosiseinstellglied 2 ist am proximalen Ende des Gehäuses 1 angeordnet und ist vom Verwender der Vorrichtung greifbar und relativ zu dem Gehäuse 1 verdrehbar.

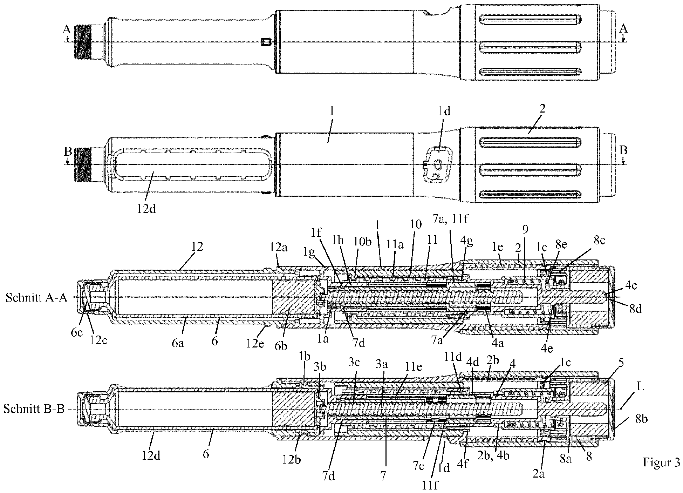

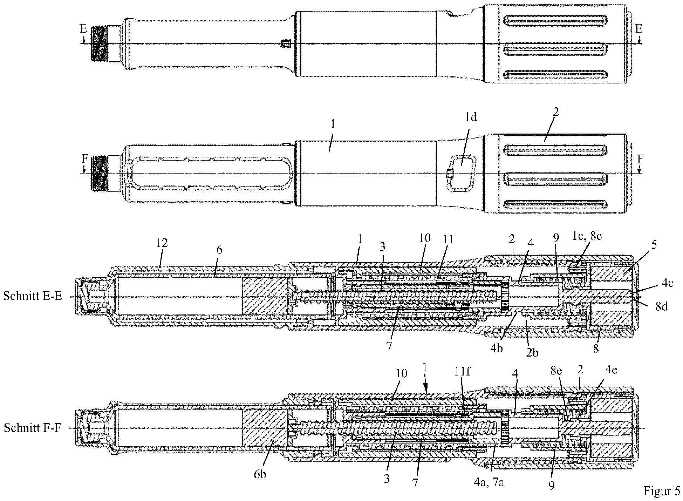

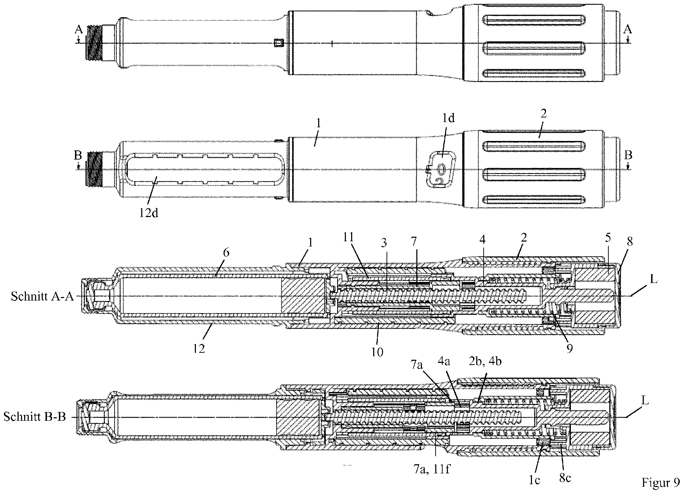

Das proximale Ende der Antriebs- und Dosiervorrichtung wird von einem Betätigungsglied 8, welches als Betätigungsknopf ausgestaltet ist, gebildet. Das Betätigungsglied 8 kann aus einer unbetätigten Position (siehe zum Beispiel Figuren 3 und 9) in eine betätigte Position (siehe zum Beispiel Figuren 5 un 10) gegen die Rücksetzfeder 9 verschoben werden, wobei hierdurch die Rücksetzfeder 9 gespannt wird. Die Rücksetzfeder 9 ist eine Wendel- oder Schraubenfeder, welche als Druckfeder wirkt und sich mit ihrem distalen Ende an dem Dosiseinstellglied 2 und mit seinem proximalen Ende an dem Betätigungsglied 8 abstützt. Beim Betätigen wird das Betätigungsglied 8 zum Beispiel mit dem Finger des Daumens der Hand, welche das Gehäuse 1 umgreift, gedrückt, wodurch die Feder 9 gespannt wird. Durch Loslassen das Betätigungsglied 8 kann die vorgespannte Feder 9 des Betätigungsglieds 8 aus der betätigten Position in die unbetätigte Position zurückschieben.

Das Betätigungsglied 8 ist relativ zu dem Dosiseinstellglied 2 entlang der Längsachse L hin und her schiebbar, nämlich zwischen der betätigten Position und der unbetätigten Position. Das Betätigungsglied 8 ist mehrteilig ausgestaltet und weist ein Verbindungsglied 8a auf, welches einen hülsenförmigen Abschnitt aufweist, der am distalen Ende durch einen nach innen ragenden Kragen verengt oder verschlossen ist. Das proximale Ende des Verbindungsglieds 8a ist mittels einer Abdeckkappe 8b verschlossen, welche ebenfalls zu dem Betätigungsglied 8a gehört und eine Auflagefläche für den Daumen zum Betätigen des Betätigungsglieds 8 bildet.

Das Betätigungsglied 8, insbesondere der hülsenförmige Abschnitt des Verbindungsglieds 8a umgibt die Antriebsfeder 5 umfangsseitig. Die Antriebsfeder 5 ist eine aus einem bandförmigen Material spiralförmig gewickelte Feder, die auch als Uhrenfeder bezeichnet werden kann. Ein erster Abschnitt, insbesondere ein erstes Ende der Antriebsfeder 5 ist an dem Betätigungsglied 8, insbesondere an dessen zylindrischem Abschnitt befestigt. Ein zweiter Abschnitt, insbesondere ein zweites Ende der Antriebsfeder 5 ist an dem Antriebsglied 4, insbesondere zwischen dessen proximalem Ende 4c und einem Kragen 4e befestigt. Zwischen dem ersten Abschnitt und dem zweiten Abschnitt weist die Antriebsfeder 5 einen dritten Abschnitt auf, der bei Änderung der Federspannung elastisch verformt wird.

Eine Drehung des Antriebsglieds 4 relativ zu dem Betätigungsglied 8 bewirkt eine Änderung der Federspannung, insbesondere eine Abnahme der Federspannung unter Abgabe der von der Feder 5 gespeicherten potentiellen Energie an das Antriebsglied 4 in Form von kinetischer, d. h. Rotationsenergie. Das Antriebsglied 4 weist einen insbesondere stab- oder stiftförmigen Abschnitt auf, der sich durch den Kragen am distalen Ende des Betätigungsglieds 8 in das Innere des Betätigungsglieds 8 und durch die Antriebsfeder 5 erstreckt. Das proximale Ende 4c verjüngt sich zum proximalen Ende der Antriebs- und Dosiervorrichtung hin, zum Beispiel kugelförmig, kegelig oder kegelstumpfförmig. Das Betätigungsglied 8, insbesondere die Abdeckkappe 8e bildet eine Kontaktfläche 8d für das proximale Ende 4c des Antriebsglieds 4.

Das Betätigungsglied 8, insbesondere das Verbindungsglied 8a weist eine sechste Kupplungsstruktur 8c in der Gestalt einer Außenverzahnung auf, die zum Beispiel an einer in distale Richtung abragenden Abragung gebildet ist. Die Abragung greift durch den Kragen, der die Innenhülse mit der Außenhülse des Dosiseinstellglieds 2 verbindet. Das Gehäuse 1, insbesondere die Außenhülse 1g weist eine fünfte Kupplungsstruktur 1c auf, die als Innenverzahnung gebildet ist und mit der sechsten Kupplungsstruktur 8c eine dritte Kupplung 1c, 8c bildet. Durch Betätigen des Betätigungsglieds 8 lässt sich die dritte Kupplung 1c, 8c einkuppeln und durch Loslassen des Betätigungsglieds 8a auskuppeln. Bei eingekuppelter dritter Kupplung 1c, 8c greift die sechste Kupplungsstruktur 8c formschlüssig in die fünfte Kupplungsstruktur 1c, wodurch das Betätigungsglied 8 und insbesondere auch das mit dem Betätigungsglied 8 drehfest verbundene Dosiseinstellglied 2, drehfest in Bezug auf das Gehäuse 1 ist. Das Betätigungsglied 8 und das Dosiseinstellglied 2 sind relativ zu dem Gehäuse 1 drehbar, wenn die dritte Kupplung 1c, 8c ausgekuppelt ist, wobei die sechste Kupplungsstruktur 8c und die fünfte Kupplungsstruktur 1c dann nicht ineinandergreifen.

An dem distalen Ende des Gehäuses 1 ist ein hülsenförmiger Produktbehälterhalter, insbesondere unlösbar befestigt, in dem ein Produktbehälter 6 aufgenommen ist. Der Produktbehälter 6 ist in dem gezeigten Beispiel eine Karpule. Der Produktbehälter 6 weist einen Behälterkörper 6a auf, der ein flüssiges, zu verabreichendes Produkt umgibt, wobei in dem Behälterkörper 6a proximal des Produkts ein verschiebbarer Kolben 6b angeordnet ist, der dichtend an der Innenwand des Behälterkörpers 6 anliegt. An dem distalen Ende des Behälterkörpers 6a ist ein Septum 6c gebildet, welches mittels einer Nadel, die an einem Gewinde 12c des Produktbehälterhalters 12 anbringbar ist, durchstochen werden kann. Zur Verschiebung des Kolbens 6b in Richtung Septum 6c wird das in dem Produktbehälter 6 enthaltende Produkt über die Nadel ausgeschüttet.

Der Produktbehälterhalter 12 weist ein Fenster 12d auf, durch welches die in dem Produktbehälter 6 enthaltene Produktmenge optisch überwacht werden kann. Der Produktbehälterhalter 12 weist eine Ausnehmung 12b auf, in die eine erste Eingriffsstruktur 1b am Innenumfang des Gehäuses 1, insbesondere der Außenhülse 1 eingeschnappt ist, wenn der Produktbehälterhalter 12 an dem Gehäuse 1 befestigt ist. Der Produktbehälterhalter 12 weist einen an seinem Außenumfang ringförmig umlaufenden Kragen 12a auf, der an dem distalen Ende des Gehäuses 1, insbesondere der Außenhülse 1g anliegt, wenn der Produktbehälterhalter 12 an dem Gehäuse 1 befestigt ist. Der Produktbehälterhalter 12 weist an seinem Außenumfang eine Nocke 12e auf, mit der eine hülsenförmige Kappe, welche über den Produktbehälterhalter 12 schiebbar ist, lösbar verschnappbar ist. Die Kappe 14 kann somit wieder abgenommen werden und dient lediglich dem Schutz einer optional angebrachten Nadel und/oder des Medikaments vor Lichteinwirkung. Zur Verschnappung mit der Nocke 12e weist die Kappe 14 eine ringförmig am Innenumfang angebrachte Ausnehmung 14a, insbesondere Nut auf.

Die Antriebs- und Dosiervorrichtung weist einen Dosisbegrenzer 13 in der Gestalt eines Ringsegments, alternativ eines Rings oder einer Mutter auf, welches an seinem Innenumfang einen Gewindeabschnitt 13b aufweist, der in ein am Außenumfang des Gehäuses 1 angeordnetes Gewinde 1e eingreift, so dass der Dosisbegrenzer 13 relativ zu dem Gehäuse 4 schraubbar ist. Der Dosisbegrenzer 13 ist in einer am Innenumfang des Dosiseinstellglieds 2 angeordneten, sich entlang der Längsachse L erstreckenden Nut 2c aufgenommen, wobei die Nutflanken den ringsegmentförmigen Dosisbegrenzer 13 seitlich einfassen. Am Außenumfang weist der Dosisbegrenzer 13 ein nutförmiges Eingriffsglied 13a, in welche eine Längsführung, die von dem Nutgrund der Nut 2c des Dosiseinstellglieds 2 abragt und sich entlang der Längsachse L erstreckt, eingreift. Sowohl durch die Nut 2c als auch durch die Längsführung ist der Dosisbegrenzer 13 relativ zu dem Dosiseinstellglied 2 drehfest, aber axial verschiebbar. An dem Dosiseinstellglied 2 oder dem Gehäuse 1 ist ein Stoppanschlag gebildet, von dem der Dosisbegrenzer 13 proportional zu der maximal aus dem Produktbehälter 6 ausschüttbaren Produktmenge beabstandet ist. Da bei der Dosiseinstellung das Dosiseinstellglied 2 relativ zu dem Gehäuse 4 verdreht und bei einer Dosisausschüttung nicht verdreht wird, kann durch den Dosisbegrenzer 13 ein Zählwert gebildet werden, welches die bereits ausgeschütteten Einzeldosen und die aktuell eingestellte Dosis addiert und sich dementsprechend immer näher an dem Stoppanschlag des Dosiseinstellglieds 2 oder des Gehäuses 1 annähert. Eine Dosiserhöhung bewirkt, dass der Dosisbegrenzer 13 zu dem Stoppanschlag hinbewegt wird. Eine Dosisverringerung bewirkt, dass der Dosisbegrenzer 13 von Stoppanschlag wegbewegt wird. Ist die in dem Produktbehälter 6 angegebene Restdosis geringer als die maximal mit der Antriebs- und Dosiervorrichtung einstellbare Dosis, gerät der Dosisbegrenzer 13 in einen Kontakt mit dem Stoppanschlag, so dass eine Verdrehung des Dosiseinstellglieds 2 relativ zu dem Gehäuse 1 in eine Drehrichtung, die eine Erhöhung der Dosis zur Folge hätte, d. h. in eine erste Drehrichtung, blockiert wird.

Das Dosisanzeigeelement 10, welches hülsenförmig ist und somit als Dosisanzeigetrommel bezeichnet werden kann, weist über seinen Außenumfang eine sich entsprechend der Steigung des Gewindes 10e wendelförmige erstreckenden Dosisskala 10b auf, die eine Vielzahl nacheinander angeordnetes Skalenwerte umfasst. In den zwei gezeigten Ausführungsformen kann mit der Antriebs- und Dosiervorrichtung eine maximale Dosis von 80 IU eingestellt werden, wobei die Skala von 0 bis 80 und die Dosiswerte in Zweierschritten angegeben sind.

Das Dosisanzeigeelement 10 weist eine in Umfangsrichtung weisende und wirkende Anschlagfläche auf, die als Nulldosisanschlag 10e bezeichnet wird. Das Dosisanzeigeelement weist eine in Umfangsrichtung weisende und wirkende Anschlagfläche auf, welche als Maximaldosisanschlag 10d bezeichnet wird.

In der Ausführungsform nach den Figuren 1 bis 6 weist das hülsenförmige Verschiebeglied 11 ein Außengewinde 11a auf, in welches das Innengewinde 10b eingreift. Das Verschiebeglied 11 weist ferner einen Nulldosisgegenanschlag 11b und einen Maximaldosisgegenanschlag 11c auf.

In der Ausführung aus den Figuren 7 bis 10 ist das Gewinde 10b ein Außengewinde, welches in einem Eingriff mit einem Innengewinde 11a der Außenhülse 1g des Gehäuses 1, oder allgemein des Gehäuses 1 ist. Das Gehäuse 1, insbesondere die Außenhülse 1g weist einen Nulldosisgegenanschlag 11b und einen Maximaldosisgegenanschlag 11c auf.

Für beide Ausführungsformen gilt, dass das Dosisanzeigeelement 10 zwischen einer Nulldosisposition und einer Maximaldosisposition hin und her schraubbar ist. In der Nulldosisposition verhindert der Nulldosisanschlag 10e im Zusammenwirken mit dem Nulldosisgegenanschlag 11b die Drehung des Dosisanzeigeelements 10 in eine zweite Drehrichtung, nämlich in eine Drehrichtung, die bewirken würde, dass eine kleinere Dosis als Null eingestellt wird. In dieser Nulldosisposition ist das Dosisanzeigeelement 10 in die entgegengesetzte, d. h. erste Drehrichtung drehbar.

In der Maximaldosisposition, verhindert der Maximaldosisanschlag 10d im Zusammenwirken mit dem Maximaldosisgegenanschlag 11c die Drehung des Dosisanzeigeelements 10 in die erste Drehrichtung, welche eine Erhöhung der Dosis über den maximal einstellbaren Wert hinaus bewirken würde. Die Drehung in die zweite Drehrichtung ist in der Maximaldosisposition möglich.

Das Gehäuse 1, insbesondere die Außenhülse 1g weist eine Zeigeeinrichtung 1d in der Gestalt eines Fensters auf, welches den Blick auf die Dosisskala 10a des Dosisanzeigeelements 10 freigibt. Das Dosisanzeigeelement 10 ist mit dem Antriebsglied 4 drehfest und axial verschiebbar verbunden. Hierfür weist das Dosisanzeigeelement 10 mindestens eine, in diesem Beispiel mehrere Führungsnuten 10c auf, die sich parallel zur Längsachse L erstrecken. In diese Führungsnuten 10c greifen Abragungen 4f am Außenumfang des Antriebsglieds 4 ein.

In den Figuren 3 und 9 werden die erste und zweite Ausführungsform in ihrem Auslieferungs- oder Ausgangszustand dargestellt, wobei die Kappe 14 entfernt wurde. Das Betätigungsglied 8 ist unbetätigt. In der Zeigeeinrichtung 1d erscheint die Dosis null, d. h., dass sich das Dosisanzeigeelement 10 in seiner Nulldosisposition befindet.

Die Antriebsfeder 5 ist mit so viel Energie vorgespannt, dass die in dem Produktbehälter 6 enthaltene Produktmenge mit der in der Feder 5 gespeicherten Energie durch Verschieben des Kolbens 6b vollständig ausgeschüttet werden kann, insbesondere in einer oder in mehreren Einzelausschüttungen, zwischen denen jeweils eine neue Dosiseinstellung stattfindet, ohne die Feder 5 zu spannen. Im Auslieferungszustand sind die erste Kupplung 4a, 7a ausgekuppelt, die zweite Kupplung 2b, 4b eingekuppelt und die dritte Kupplung 1c, 8c ausgekuppelt. Die Antriebsfeder 5 ist kinematisch zwischen das Antriebsglied 4 und das Dosiseinstellglied 2 geschaltet. Die Kupplung 2b, 4b verhindert, dass das Antriebsglied 4 relativ zu dem Dosiseinstellglied 2 gedreht wird.

Zum Einstellen der auszuschüttenden Produktdosis wird das Dosiseinstellglied 2 relativ zu dem Gehäuse 1 in eine erste Drehrichtung gedreht, wodurch das Antriebsglied 4, die Feder 5 und das Dosisanzeigeelement 10 ebenfalls mitgedreht werden. Durch die Drehung wird das Dosisanzeigeelement 10 von dem Nulldosisgegenanschlag 11b weggeschraubt, wobei der zwischen dem Nulldosisanschlag 10e und dem Nulldosisgegenanschlag 11b entlang der Schraubkurve gemessene Abstand proportional zu der auszuschüttenden Produktdosis ist. Durch die Zeigeeinrichtung 1d kann die aktuell eingestellte Dosis in IU abgelesen werden. Sollte die Dosis versehentlich zu hoch eingestellt worden sein, kann das Dosiseinstellglied 2 durch Drehen in die entgegengesetzte Drehrichtung, d. h. in die zweite Drehrichtung relativ zu dem Gehäuse 1 gedreht werden, wodurch der Abstand zwischen dem Nulldosisanschlag und dem Maximaldosisanschlag abnimmt und die ablesbare Dosis verringert wird.

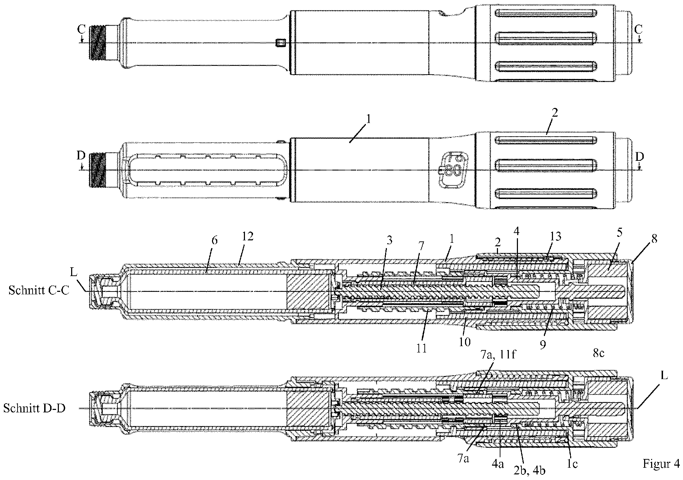

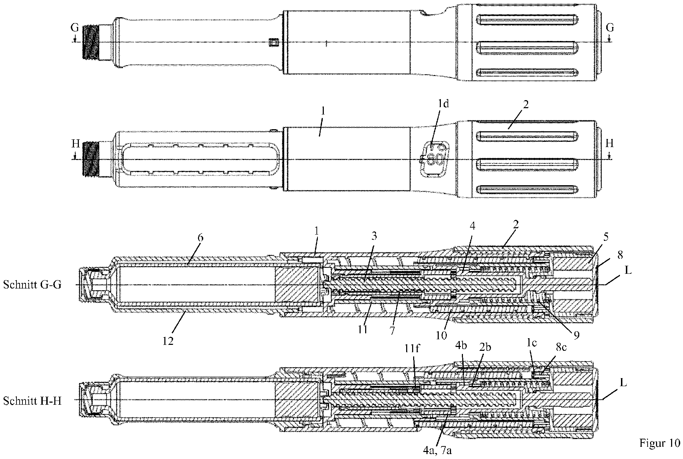

Für die Produktausschüttung wird das Betätigungsglied 8 zum Beispiel mit dem Daumen der Hand, welche das Gehäuse 1 umgreift, aus einer unbetätigten Position (zum Beispiel Figuren 4 und 9) in eine betätigte Position (zum Beispiel Figuren 5 und 10) gedrückt, wodurch die Rücksetzfeder 9 gespannt wird. Während der Bewegung des Betätigungsglieds 8 aus der unbetätigten Position in die betätigte Position wird zunächst die dritte Kupplung 1c, 8c eingekuppelt, so dass die Antriebsfeder 5 kinematisch zwischen das Gehäuse 1 und das Antriebsglied 4 geschaltet ist. Das Dosiseinstellglied 2 ist dann auch verdrehgesichert in Bezug auf das Gehäuse 1. Wird das Betätigungsglied 8 weiter in die distale Richtung zu der betätigten Position gedrückt, wird die erste Kupplung 4a, 7a eingekuppelt, wodurch das Rotationsglied 7 in Bezug auf das Antriebsglied 4 verdrehfest ist. Weiteres Verschieben des Betätigungsglieds 8 zu seiner betätigten Positio hin bewirkt, dass die zweite Kupplung 2b, 4b ausgekuppelt wird, so dass das Drehmoment der Antriebsfeder 5 über die vierte Kupplung 7a, 11f in das Gehäuse 1 geleitet wird, wodurch sich die Antriebsfeder 5 noch nicht entspannen kann. Erst, wenn die vierte Kupplung 7a, 11f mit dem Erreichen der betätigten Position des Betätigungsglieds 8 ausgekuppelt wird, kann die Antriebsfeder 5, die sich mit ihrem ersten Ende an dem Gehäuse 1 abstützt, die in ihr gespeicherte Federenergie in Form von Rotationsenergie über das Antriebsglied 4 an das Rotationsglied 7 abgegeben, wodurch sich das Antriebsglied 4 in die zweite Drehrichtung dreht. Dabei dreht sich auch das Rotationsglied 7 in die zweite Drehrichtung, wodurch das Abtriebsglied 3 ebenfalls in die zweite Drehrichtung gedreht wird und sich dadurch an dem Innengewinde 1a in die distale Richtung schraubt, wodurch der Kolben 6e in die distale Richtung bewegt wird und das in dem Produktbehälter 6 enthaltene Produkt ausschüttet. Aufgrund der drehfesten Verbindung zwischen dem Antriebsglied 4 und dem Dosiseinstellglied 10 wird gleichzeitig das Dosisanzeigeelement 10 in seine Nulldosisposition zurückgeschraubt. Wenn der Nulldosisanschlag 10e an dem Nulldosisgegenanschlag 11b anschlägt, ist die eingestellte Dosis vollständig ausgeschüttet, wobei durch das Anschlagen des Nulldosisanschlags 10e an dem Nulldosisgegenanschlag 11b das Dosisanzeigeelement 10 hinsichtlich seiner Drehung gestoppt wird, wodurch auch das Antriebsglied 4 hinsichtlich seiner Drehung in die zweite Drehrichtung gestoppt wird. Diese Situation ist zum Beispiel in Figur 5 dargestellt. Wenn der Benutzer das Betätigungsglied 8 los lässt, setzt die Rücksetzfeder 9 das Betätigungsglied 8 in seine unbetätigte Position zurück, wobei die erste Kupplung 4a, 7a, die zweite Kupplung 2b, 4b, die dritte Kupplung 1c, 8c und die vierte Kupplung 7a, 11f in ihre Ausgangslagen zurückgestellt werden. Durch Drehen des Dosiseinstellglieds 2 in die erste Drehrichtung kann nun wieder eine auszuschüttende Dosis eingestellt werden, die wieder durch Betätigen des Betätigungsglieds 8 ausgeschüttet werden kann, usw. Wenn in dem Produktbehälter 6 eine Produktmenge enthalten sein sollte, die geringer als die maximal mit der Vorrichtung ausschüttbaren Dosis, in diesem Beispiel kleiner als 80 IU, ist, schlägt der Dosisbegrenzer 13 an seinem Endanschlag an, wenn das Dosiseinstellglied 2 in die erste Drehrichtung gedreht wird, bevor der Maximaldosisanschlag 10d an dem Maximaldosisgegenanschlag 11c anschlägt. Hierdurch wird verhindert, dass sich der Verwender der Vorrichtung weniger Produkt injizieren kann als er mit der Vorrichtung eingestellt hat.

**Bezugszeichenliste**

| | | | |
|---|---|---|---|
| 1 | Gehäuse | 8a | Verbindungsglied |
| 1a | Gewinde / Innengewinde | 8b | Abdeckkappe |
| 1b | erste Eingriffsstruktur | 8c | sechste Kupplungsstruktur |
| 1c | fünfte Kupplungsstruktur | 8d | Kontaktfläche |
| 1d | Zeigeeinrichtung | 8e | Eingriffsnocke |
| 1e | Außengewinde | | |
| 1f | Abragung | 9 | Rücksetzfeder |
| 1g | Außenhülse | | |
| 1h | Innenhülse | 10 | Dosisanzeigeelement / Dosisanzeigetrommel |
| 2 | Dosiseinstellglied | 10a | Dosisskala |
| 2a | Abragung | 10b | Gewinde |
| 2b | dritte Kupplungsstruktur | 10c | Führungsnut |
| 2c | Nut | 10d | Maximaldosisanschlag |
| | | 10e | Nulldosisanschlag |
| 3 | Abtriebsglied | | |
| 3a | Gewindestange | 11 | Verschiebeglied |
| 3b | Flansch | 11a | Außengewinde |
| 3c | Gewinde / Außengewinde | 11b | Nulldosisgegenanschlag |
| 3d | Führung / Führungsnut | 11c | Maximaldosisgegenanschlag |
| | | 11d | Ringnut |
| 4 | Antriebsglied | 11e | Längsführung |
| 4a | erste Kupplungsstruktur | 11f | Innenverzahnung / siebte Kupplungsstruktur |
| 4b | vierte Kupplungsstruktur | | |
| 4c | proximales Ende | | |
| 4d | Raststruktur / Klicker | 12 | Produktbehälterhalter |
| 4e | Kragen | 12a | Kragen |
| 4f | Abragung / Steg | 12b | Ausnehmung |
| 4g | Abragung / Nocke | 12c | Gewinde |
| | | 12d | Fenster |
| 5 | Antriebsfeder | 12e | Nocke |
| 6 | Produktbehälter / Karpule | 13 | Begrenzer / Dosisbegrenzer |
| 6a | Behälterkörper | 13a | Nut |
| 6b | Kolben | 13b | Gewinde(-abschnitt) |
| 6c | Septum | 14 | Kappe |
| 7 | Rotationsglied | 14a | Ausnehmung |
| 7a | zweite Kupplungsstruktur | | |
| 7b | Führung / Eingriffsglied | 4a, 7a | erste Kupplung |
| 7c | Rastmittel / Rastarm | 2b, 4b | zweite Kupplung |
| 7d | Nut | 1c, 8c 7a, 11f | dritte Kupplung vierte Kupplung |
| 8 | Betätigungsglied / Betätigungsknopf | L | Längsachse |

## Patentansprüche

1. Antriebs- und Dosiervorrichtung für eine Injektionsvorrichtung zur Ausschüttung eines flüssigen Produkts, die Antriebs- und Dosiervorrichtung umfassend:
a) ein Gehäuse (1),
b) ein Dosiseinstellglied (2), welches für die Einstellung einer auszuschüttenden Produktdosis relativ zu dem Gehäuse (1) drehbar ist,
c) ein in dem Gehäuse (1) aufgenommenes Abtriebsglied (3),
d) ein Antriebsglied (4), welches relativ zu dem Gehäuse (1) drehbar ist und während der Produktausschüttung so mit dem Abtriebsglied (3) gekoppelt ist, dass eine Drehung des Antriebsglieds (4) bewirkt, dass das Abtriebsglied (3) relativ zu dem Gehäuse (1) in die distale Richtung bewegt wird,
**dadurch gekennzeichnet, dass** die Antriebs- und Dosiervorrichtung weiter
e) eine vorgespannte Antriebsfeder (5) umfasst, die während der Einstellung der Produktdosis zwischen das Dosiseinstellglied (2) und das Antriebsglied (4) geschaltet ist, wobei das Dosiseinstellglied (2) während der Einstellung der Produktdosis verdrehfest mit dem Antriebsglied (4) gekoppelt ist, so dass eine Drehung des Antriebsglieds (4) relativ zu dem Dosiseinstellglied (2) verhindert wird,
f) wobei das Dosiseinstellglied (2) während der Produktausschüttung relativ zu dem Gehäuse (1) verdrehfest ist.

2. Antriebs- und Dosiervorrichtung nach Anspruch 1, **dadurch gekennzeichnet, dass** die Antriebsfeder (5) so stark vorgespannt ist, dass die in der Antriebsfeder (5) gespeicherte Federenergie ausreicht, das in einem an der Antriebs- und Dosiervorrichtung befestigten oder befestigbaren Produktbehälter (6) enthaltene Produkt vollständig auszuschütten.

3. Antriebs- und Dosiervorrichtung nach einem der vorhergehenden Ansprüche, **dadurch gekennzeichnet, dass** das Dosiseinstellglied (2) und das Antriebsglied (4) während der Einstellung der Produktdosis relativ zu dem Abtriebsglied (3) und/oder zu dem Gehäuse (1) in eine erste Drehrichtung, wodurch eine Erhöhung der auszuschüttenden Produktdosis bewirkt wird, und in eine zweite Drehrichtung, wodurch eine Verringerung der auszuschüttenden Produktdosis bewirkt wird, drehbar sind.

4. Antriebs- und Dosiervorrichtung nach einem der vorhergehenden Ansprüche, **dadurch gekennzeichnet, dass** kinematisch zwischen dem Antriebsglied (4) und dem Abtriebsglied (3) ein Rotationsglied (7) angeordnet ist, wobei entweder
- das Rotationsglied (7) in einem Gewindeeingriff mit einem Gewinde (3c) des Abtriebsglieds (3) ist und das Abtriebsglied (3) mit einer gehäusefesten Führung der Antriebs- und Dosiervorrichtung in einem Eingriff ist, so dass eine Drehung des Rotationsglieds (7) eine Bewegung des Abtriebsglieds (3) in die distale Richtung bewirkt,
oder
- ein gehäusefestes Gewinde (1a) in einem Gewindeeingriff mit einem Gewinde (3c) des Abtriebsglieds (3) ist und das Abtriebsglied (3) mit einer Führung (7b) des Rotationsglieds (7) in einem Eingriff ist, so dass eine Drehung des Rotationsglieds (7) eine Schraubbewegung des Abtriebsglieds (3) in die distale Richtung bewirkt.

5. Antriebs- und Dosiervorrichtung nach einem der vorhergehenden Ansprüche, **dadurch gekennzeichnet, dass** eine Drehung des Antriebsglieds (4) relativ zu dem Gehäuse (1) und/oder dem Dosiseinstellglied (2) bewirkt, dass das Abtriebsglied (3) relativ zu dem Gehäuse (1) in die distale Richtung bewegt wird.

6. Antriebs- und Dosiervorrichtung nach einem der vorhergehenden Ansprüche, **gekennzeichnet durch** ein Betätigungsglied (8), insbesondere einen am proximalen Ende der Antriebs- und Dosiervorrichtung gebildeten Betätigungsknopf, welches aus einer unbetätigten Position, die es während der Einstellung der auszuschüttenden Produktdosis einnimmt, in eine betätigte Position, die es während der Produktausschüttung einnimmt, verschiebbar ist, wobei vorzugsweise eine Rücksetzfeder (9) vorgesehen ist, die so auf das Betätigungsglied (8) wirkt, dass die Rücksetzfeder (9) gespannt wird, wenn das Betätigungsglied (8) aus seiner unbetätigten Position in seine betätigte Position verschoben wird, und insbesondere dass das Betätigungsglied (8) mittels der Rücksetzfeder (9) aus seiner betätigten Position in seine unbetätigte Position verschiebbar ist.

7. Antriebs- und Dosiervorrichtung nach einem der vorhergehenden Ansprüche, **gekennzeichnet durch** eine erste Kupplung (4a, 7a), welche zwischen dem Antriebsglied (4) und dem Abtriebsglied (3) angeordnet ist, wobei die erste Kupplung (4a, 7a) während der Einstellung der Produktdosis ausgekuppelt ist und hierdurch dem Antriebsglied (4) erlaubt, relativ zu dem Abtriebsglied (3) verdreht zu werden, und während der Produktausschüttung eingekuppelt ist.

8. Antriebs- und Dosiervorrichtung nach einem der vorhergehenden Ansprüche, **gekennzeichnet durch** eine zweite Kupplung (2b, 4b), welche zwischen dem Dosiseinstellglied (2) und dem Antriebsglied (4) angeordnet ist, wobei die zweite Kupplung (2b, 4b) während der Einstellung der Produktdosis eingekuppelt ist, wodurch das Dosiseinstellglied (2) und das Antriebsglied (4) verdrehgesichert sind, und während der Produktausschüttung ausgekuppelt ist, wodurch das Antriebsglied (4) mittels der vorgespannten Antriebsfeder (5) relativ zu dem Dosiseinstellglied (2) drehbar ist.

9. Antriebs- und Dosiervorrichtung nach einem der vorhergehenden Ansprüche, **gekennzeichnet durch** eine dritte Kupplung (1c, 8c), welche zwischen dem Dosiseinstellglied (2) und dem Gehäuse (1) angeordnet ist, wobei die dritte Kupplung (1c, 8c) während der Einstellung der Produktdosis ausgekuppelt ist, wodurch das Dosiseinstellglied (2) relativ zu dem Gehäuse (1) verdrehbar ist, und während der Produktausschüttung eingekuppelt ist, wodurch das Dosiseinstellglied (2) relativ zu dem Gehäuse (1) drehfest ist.

10. Antriebs- und Dosiervorrichtung nach dem vorhergehenden Anspruch, **dadurch gekennzeichnet, dass** die dritte Kupplung (1c, 8c) zwischen dem Betätigungsglied (8) und dem Gehäuse (1) gebildet ist, wobei das Betätigungsglied (8) und das Dosiseinstellglied (2), insbesondere während der Einstellung der Produktdosis und während der Produktausschüttung, verdrehfest verbunden sind.

11. Antriebs- und Dosiervorrichtung nach einem der vorhergehenden Ansprüche, **dadurch gekennzeichnet, dass** die Antriebsfeder (5) einen ersten Abschnitt, mit dem sich die Antriebsfeder (5) während der Einstellung der Produktdosis in Bezug auf das Dosiseinstellglied (2) oder während der Produktausschüttung in Bezug auf das Gehäuse (1) verdrehfest abstützt, einen zweiten Abschnitt, mit dem sich die Antriebsfeder (5) in Bezug auf das Antriebsglied (4) verdrehfest abstützt, und einen dritten Abschnitt, der zwischen dem ersten Abschnitt und dem zweiten Abschnitt angeordnet ist und sich bei einer Änderung der Federspannung der Antriebsfeder (5) elastisch verformt, aufweist, wobei bevorzugt ist, dass der erste Abschnitt und der zweite Abschnitt relativ zu dem Gehäuse (1) verdreht werden und insbesondere zueinander stillstehen, wenn das Dosiseinstellglied (2) für die Dosiseinstellung gedreht wird.

12. Antriebs- und Dosiervorrichtung nach einem der vorhergehenden Ansprüche, **gekennzeichnet durch** ein Dosisanzeigeelement (10), welches an seinem Außenumfang eine Dosisskala (10a) aufweist und verdrehfest mit dem Antriebsglied (4) verbunden ist, so dass das Dosisanzeigeelement (10) die Drehbewegungen des Antriebsglieds (4) mitmacht, wobei das Gehäuse (1) eine Zeigeeinrichtung (1d), insbesondere ein Fenster aufweist, durch welche ein Skalenwert der Dosisskala (10a), welcher mit der eingestellten Dosis korrespondiert, ablesbar ist.

13. Antriebs- und Dosiervorrichtung nach einem der vorhergehenden Ansprüche, **dadurch gekennzeichnet, dass** die Antriebsfeder (5) in dem Betätigungsglied (8) aufgenommen ist und mit einem ersten Abschnitt an dem Betätigungsglied (8) anliegt und hierdurch mittelbar an dem Dosiseinstellglied (2) und/oder dem Gehäuse (1) abgestützt ist.

14. Antriebs- und Dosiervorrichtung nach einem der vorhergehenden Ansprüche, **dadurch gekennzeichnet, dass** zwischen dem Antriebsglied (4) und dem Betätigungsglied (8) eine Einrichtung (4c, 8d) angeordnet ist, welche insbesondere die Reibung zwischen dem Betätigungsglied (8) und dem Antriebsglied (4) verringert, wenn sich das Antriebsglied (4) während der Produktausschüttung relativ zu dem Betätigungsglied (8) dreht, wobei die Einrichtung (4c, 8d) vorzugsweise dergestalt ist, dass sich das proximale Ende (4c) des Antriebsglieds (4) zu einer Kontaktfläche (8d) des Betätigungsglieds (8) hin verjüngt, wobei zwischen dem proximalen Ende (4c) des Antriebsglieds (4) und der Kontaktfläche (8d) ein Spalt besteht, wenn das Betätigungsglied (8) in seiner unbetätigten Position ist, und das proximale Ende (4c) des Antriebsglieds (4) an der Kontaktfläche (8d) anliegt, wenn das Betätigungsglied (8) in seiner betätigten Position ist.

## Claims

1. A drive and dosing device for an injection device for discharge of a liquid product, the drive and dosing device comprising:
a) a housing (1);
b) a dose selection member (2), which can be rotated relative to the housing (1) for the selection of a product dose to be discharged;
c) a driven member (3) accommodated in the housing (1);
d) a driving member (4), which can be rotated relative to the housing (1), and during the product discharge is coupled to the driven member (3) so that a rotation of the driving member (4) causes the driven member (3) to be moved in the distal direction relative to the housing (1); **characterized in that** the drive and dosing device further comprises
e) a pretensioned drive spring (5), which is connected between the dose selection member (2) and the driving member (4) during the selection of the product dose, where the dose selection member (2) is coupled non-rotatably to the driving member (4) during the selection of the product dose, such that a rotation of the driving member (4) relative to the dose selection member (2) is prevented,
f) wherein the dose selection member (2) is non-rotatable relative to the housing (1) during the product discharge.

2. The drive and dosing device according to claim 1, wherein the drive spring (5) is pretensioned such that spring energy stored in the drive spring (5) is sufficient to discharge completely the liquid product comprised in a product container (6) attached to the drive and dosing device.

3. The drive and dosing device according to any one of the preceding claims,
wherein during the selection of the product dose, the dose selection member (2) and the driving member (4) are rotatable relative to the driven member (3) and/or the housing (1) in a first direction of rotation to increase the product dose to be discharged, and in a second direction of rotation to reduce the product dose to be discharged.

4. The drive and dosing device according to the preceding claim, further comprising a rotation member (7) operably connected between the driving member (4) and the driven member (3), wherein either the rotation member (7) is in a threaded engagement with a thread (3c) of the driven member (3) and the driven member (3) is in an engagement with a guide of the drive and dosing device that is affixed to the housing such that a rotation of the rotation member (7) causes a movement of the driven member (3) in the distal direction, or wherein a thread (1a) that is affixed to the housing is in a threaded engagement with a thread (3c) of the driven member (3) and the driven member (3) is in engagement with a guide (7b) of the rotation member (7) such that a rotation of the rotation member (7) causes a screwing motion of the driven member (3) in the distal direction.

5. The drive and dosing device according to any one of the preceding claims,
wherein a rotation of the driving member (4) relative to the housing (1) and/or the dose selection member (2) causes the driven member (3) to be moved in the distal direction relative to the housing (1).

6. The drive and dosing device according to any one of the preceding claims, further comprising an actuation member (8), preferably an actuation knob arranged at a proximal end of the drive and dosing device, wherein the actuation member is movable from an unactuated position, which it takes during the selection of the product dose to be discharged, to an actuated position, which it takes during the product discharge, preferably further comprising a reset spring (9), wherein the reset spring (9) is tensed when the actuation member (8) is moved from its unactuated position to its actuated position, and wherein the tensed reset spring (9) moves the actuation member (8) from the actuated position to the unactuated position.

7. The drive and dosing device according to any one of the preceding claims, further comprising a first coupling (4a, 7a) between the driving member (4) and the driven member (3), wherein the first coupling (4a, 7a) is uncoupled during the selection of the product dose thereby enabling the driving member (4) to rotate relative to the driven member (3), and wherein the first coupling (4a, 7a) is coupled during product discharge..

8. The drive and dosing device according to any one of the preceding claims, further comprising a second coupling (2b, 4b) arranged between the dose selection member (2) and the driving member (4), where the second coupling (2b, 4b) is coupled during the selection of the product dose such that the dose selection member (2) cannot rotate relative to the driving member (4), and where the second coupling (2b, 4b) is uncoupled during product discharge such that the driving member (4) may rotate relative to the dose selection member (2) by means of the pretensioned drive spring (5).

9. The drive and dosing device according to any one of the preceding claims, further comprising a third coupling (1c, 8c) between the dose selection member (2) and the housing (1), where the third coupling (1c, 8c) is uncoupled during the selection of the product dose, whereby the dose selection member (2) can be rotated relative to the housing (1), and is coupled during the product discharge, whereby the dose selection member (2) cannot rotate relative to the housing (1).

10. The drive and dosing device according to the preceding claims, wherein the third coupling (1c, 8c) is between the actuation member (8) and the housing (1), wherein the actuation member (8) and the dose selection member (2) are non-rotatably coupled during the selection of the product dose and during the product discharge.

11. The drive and dosing device according to any one of the preceding claims,
wherein the drive spring (5) includes a first segment, by which the drive spring (5) is braced non-rotatably with respect to the dose selection member (2) during the selection of the product dose or with respect to the housing (1) during the product discharge, a second segment, by which the drive spring (5) is braced non-rotatably with respect to the driving member(4), and a third segment, which is disposed between the first segment and the second segment and elastically deforms upon a change of the spring tension of the drive spring (5), wherein preferably the first segment and the second segment are rotated relative to the housing (1) and are stationary with respect to each other when the dose selection member (2) is rotated for the dose selection.

12. The drive and dosing device according to any one of the preceding claims, further comprising a dose display element (10) comprising a dose scale (10a) on its outer circumference, wherein the display element (10) is connected non-rotatably to the driving member (4) such that the dose display element (10) follows the rotary movements of the driving member (4), and wherein the housing (1) comprises an indicator device (1d) through which a scale value of the dose scale (10a) corresponding to a selected dose can be read.

13. The drive and dosing device according to any one of the preceding claims,
wherein the drive spring (5) is accommodated in the actuation member (8) and couples to the actuation member (8) via a first segment such that the drive spring (5) is supported indirectly at the dose selection member (2) and/or the housing (1).

14. The drive and dosing device according to any one of the preceding claims, wherein a contact (4c, 8d) is arranged between the driving member (4) and the actuation member (8), which contact is adapted to reduce friction between the actuation member (8) and the driving member (4) when the driving member (4) rotates relative to the actuation member (8) during the product discharge, wherein preferably the contact (4c, 8d) comprises a proximal end (4c) of the driving member (4) tapering towards a contact surface (8d) of the actuation member (8), and wherein a gap is defined between the proximal end (4c) and the contact surface (8d) when the actuation member (8) is in its unactuated position, and wherein the proximal end (4c) fits closely to the contact surface (8d) when the actuation member (8) is in its actuated position.

## Revendications

1. Dispositif d'entraînement et de dosage pour un dispositif d'injection servant à distribuer un produit liquide, le dispositif d'entraînement et de dosage comprenant :
a) un boîtier (1),
b) un organe de réglage de dose (2), lequel peut tourner par rapport au boîtier (1) pour le réglage d'une dose de produit à distribuer,
c) un organe de sortie (3) logé dans le boîtier (1),
d) un organe d'entraînement (4), qui peut tourner par rapport au boîtier (1) et est couplé à l'organe de sortie (3) pendant la distribution de produit de telle sorte qu'une rotation de l'organe d'entraînement (4) a pour effet que l'organe de sortie (3) est déplacé dans la direction distale par rapport au boîtier (1),
**caractérisé en ce que** le dispositif d'entraînement et de dosage comprend par ailleurs
e) un ressort d'entraînement (5) précontraint, qui est connecté pendant le réglage de la dose de produit entre l'organe de réglage de dose (2) et l'organe d'entraînement (4), l'organe de réglage de dose (2) étant couplé de manière solidaire en rotation à l'organe d'entraînement (4) pendant le réglage de la dose de produit de sorte qu'une rotation de l'organe d'entraînement (4) par rapport à l'organe de réglage de dose (2) soit empêchée,
f) dans lequel l'organe de réglage de dose (2) est solidaire en rotation par rapport au boîtier (1) pendant la distribution de produit.

2. Dispositif d'entraînement et de dosage selon la revendication 1, **caractérisé en ce que** le ressort d'entraînement (5) est précontraint si fortement que l'énergie de ressort stockée dans le ressort d'entraînement (5) suffit pour réaliser une distribution en totalité du produit contenu dans un contenant de produit (6) fixé ou pouvant être fixé au niveau du dispositif d'entraînement et de dosage.

3. Dispositif d'entraînement et de dosage selon l'une quelconque des revendications précédentes, **caractérisé en ce que** l'organe de réglage de dose (2) et l'organe d'entraînement (4) peuvent être tournés pendant le réglage de la dose de produit par rapport à l'organe de sortie (3) et/ou au boîtier (1) dans une première direction de rotation, ce qui a pour effet d'augmenter la dose de produit à distribuer, et dans une seconde direction de rotation, ce qui a pour effet de réduire la dose de produit à distribuer.

4. Dispositif d'entraînement et de dosage selon l'une quelconque des revendications précédentes, **caractérisé en ce qu'**un organe de rotation (7) est disposé de manière cinématique entre l'organe d'entraînement (4) et l'organe de sortie (3), dans lequel soit
- l'organe de rotation (7) est en prise par filetage avec un filetage (3c) de l'organe de sortie (3) et l'organe de sortie (3) est en prise avec un guidage solidaire du boîtier du dispositif d'entraînement et de dosage de sorte qu'une rotation de l'organe de rotation (7) ait pour effet un déplacement de l'organe de sortie (3) dans la direction distale,
soit
- un filetage (la) solidaire du boîtier est en prise par filetage avec un filetage (3c) de l'organe de sortie (3) et l'organe de sortie (3) est en prise avec un guidage (7b) de l'organe de rotation (7) de sorte qu'une rotation de l'organe de rotation (7) ait pour effet un déplacement de vissage de l'organe de sortie (3) dans la direction distale.

5. Dispositif d'entraînement et de dosage selon l'une quelconque des revendications précédentes, **caractérisé en ce qu'**une rotation de l'organe d'entraînement (4) par rapport au boîtier (1) et/ou à l'organe de réglage de dose (2) a pour effet que l'organe de sortie (3) est déplacé par rapport au boîtier (1) dans la direction distale.

6. Dispositif d'entraînement et de dosage selon l'une quelconque des revendications précédentes, **caractérisé par** un organe d'actionnement (8), en particulier un bouton d'actionnement formé au niveau de l'extrémité proximale du dispositif d'entraînement et de dosage, lequel peut être coulissé depuis une position non actionnée, qu'il adopte pendant le réglage de la dose de produit à distribuer, dans une position actionnée, qu'il adopte pendant la distribution de produit, dans lequel de préférence un ressort de réinitialisation (9) est prévu, qui agit de telle sorte sur l'organe d'actionnement (8) que le ressort de réinitialisation (9) est tendu quand l'organe d'actionnement (8) est coulissé depuis sa position non actionnée dans sa position actionnée, et en particulier que l'organe d'actionnement (8) peut être coulissé au moyen du ressort de réinitialisation (9) depuis sa position actionnée dans sa position non actionnée.

7. Dispositif d'entraînement et de dosage selon l'une quelconque des revendications précédentes, **caractérisé par** un premier couplage (4a, 7a), qui est disposé entre l'organe d'entraînement (4) et l'organe de sortie (3), dans lequel le premier couplage (4a, 7a) est désaccouplé pendant le réglage de la dose de produit et permet ainsi à l'organe d'entraînement (4) d'être tourné par rapport à l'organe de sortie (3), et est accouplé pendant la distribution de produit.

8. Dispositif d'entraînement et de dosage selon l'une quelconque des revendications précédentes, **caractérisé par** un deuxième couplage (2b, 4b), qui est disposé entre l'organe de réglage de dose (2) et l'organe d'entraînement (4), dans lequel le deuxième couplage (2b, 4b) est accouplé pendant le réglage de la dose de produit, ce qui permet de bloquer en rotation l'organe de réglage de dose (2) et l'organe d'entraînement (4), et est désaccouplé pendant la distribution de produit, ce qui permet de faire tourner l'organe d'entraînement (4) au moyen du ressort d'entraînement (5) précontraint par rapport à l'organe de réglage de dose (2).

9. Dispositif d'entraînement et de dosage selon l'une quelconque des revendications précédentes, **caractérisé par** un troisième couplage (1c, 8c), qui est disposé entre l'organe de réglage de dose (2) et le boîtier (1), dans lequel le troisième couplage (1c, 8c) est désaccouplé pendant le réglage de la dose de produit, ce qui permet de faire tourner l'organe de réglage de dose (2) par rapport au boîtier (1), et est accouplé pendant la distribution de produit, ce qui permet de rendre solidaire en rotation l'organe de réglage de dose (2) par rapport au boîtier (1).

10. Dispositif d'entraînement et de dosage selon la revendication précédentes, **caractérisé en ce que** le troisième couplage (1c, 8c) est formé entre l'organe d'actionnement (8) et le boîtier (1), dans lequel l'organe d'actionnement (8) et l'organe de réglage de dose (2) sont reliés de manière solidaire en rotation, en particulier pendant le réglage de la dose de produit et pendant la distribution de produit.

11. Dispositif d'entraînement et de dosage selon l'une quelconque des revendications précédentes, **caractérisé en ce que** le ressort d'entraînement (5) présente une première section, par laquelle le ressort d'entraînement (5) prend appui de manière solidaire en rotation pendant le réglage de la dose de produit par rapport à l'organe de réglage de dose (2) ou pendant la distribution de produit par rapport au boîtier (1), une deuxième section, par laquelle le ressort d'entraînement (5) prend appui de manière solidaire en rotation par rapport à l'organe d'entraînement (4), et une troisième section, qui est disposée entre la première section et la deuxième section et qui se déforme de manière élastique dans le cas d'une modification de la tension de ressort du ressort d'entraînement (5), dans lequel de préférence la première section et la deuxième section sont contraintes en torsion par rapport au boîtier (1) et en particulier sont à l'arrêt quand l'organe de réglage de dose (2) est tourné pour le réglage de dose.

12. Dispositif d'entraînement et de dosage selon l'une quelconque des revendications précédentes, **caractérisé par** un élément d'affichage de dose (10), qui présente au niveau de sa périphérie extérieure une graduation de dose (10a) et qui est relié de manière solidaire en rotation à l'organe d'entraînement (4) de sorte que l'élément d'affichage de dose (10) accompagne les déplacements de rotation de l'organe d'entraînement (4), dans lequel le boîtier (1) présente un système indicateur (1d), en particulier une fenêtre, par lequel une valeur d'échelle de la graduation de dose (10a), qui correspond à la dose réglée, peut être lue.

13. Dispositif d'entraînement et de dosage selon l'une quelconque des revendications précédentes, **caractérisé en ce que** le ressort d'entraînement (5) est logé dans l'organe d'actionnement (8) et repose par une première section au niveau de l'organe d'actionnement (8) et est soutenu ce faisant indirectement au niveau de l'organe de réglage de dose (2) et/ou du boîtier (1).

14. Dispositif d'entraînement et de dosage selon l'une quelconque des revendications précédentes, **caractérisé en ce qu'**est disposé entre l'organe d'entraînement (4) et l'organe d'actionnement (8) un système (4c, 8d), lequel réduit en particulier le frottement entre l'organe d'actionnement (8) et l'organe d'entraînement (4) quand l'organe d'entraînement (4) tourne par rapport à l'organe d'actionnement (8) pendant la distribution de produit, dans lequel le système (4c, 8d) est de préférence tel que l'extrémité proximale (4c) de l'organe d'entraînement (4) se rétrécit en direction d'une surface de contact (8d) de l'organe d'actionnement (8), dans lequel une fente existe entre l'extrémité proximale (4c) de l'organe d'entraînement (4) et la surface de contact (8d) quand l'organe d'actionnement (8) est dans sa position non actionnée, et l'extrémité proximale (4c) de l'organe d'entraînement (4) repose au niveau de la surface de contact (8d) quand l'organe d'actionnement (8) est dans sa position actionnée.
